# EUROPEAN PATENT APPLICATION

(11) **EP 3 922 993 A1**
(43) Date of publication of application: **15.12.2021**
(21) Application number: 20179763.6
(22) Date of filing: 12.06.2020
(51) Int. Cl.: G01N 33/569, C07K 16/28, C07K 16/40, G01N 33/573, A61K 38/00, A61K 45/00, A61P 9/00, A61P 31/00

(54) **DPP3 IN PATIENTS INFECTED WITH CORONAVIRUS**

(71) Applicant: 4TEEN4 Pharmaceuticals GmbH, 16761 Hennigsdorf (DE)
(72) Inventor: Bergmann, Andreas, 13465 Berlin (DE)
(74) Representative: Kilger, Ute

(57) **Abstract**

Subject matter of the present invention is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus, the method comprising:
• determining the level of dipeptidyl peptidase 3 (DPP3) in a sample of bodily fluid of said patient,
• comparing said level of determined DPP3 to a pre-determined threshold, and
• correlating said level of determined DPP3 with the risk of life-threatening deterioration or an adverse event, or
• correlating said level of determined DPP3 with the severity, or
• correlating said level of determined DPP3 with the success of a therapy or intervention, or
• correlating said level of DPP3 with a certain therapy or intervention, or
• correlating said level of DPP3 with the management of said patient.

Subject matter of the present invention is an inhibitor of the activity of DPP3 for use in therapy or intervention in a patient infected with a coronavirus.

SEQUENCE LISTING

<110>  4TEEN4 Pharmaceuticals GmbH

<120>  DPP3 in patients infected with coronavirus

<130>  T75121EP01

<160>  13

<170>  PatentIn version 3.5

<210>  1
<211>  737
<212>  PRT
<213>  Homo sapiens

<400>  1

Met Ala Asp Thr Gln Tyr Ile Leu Pro Asn Asp Ile Gly Val Ser Ser
1               5                   10                  15

Leu Asp Cys Arg Glu Ala Phe Arg Leu Leu Ser Pro Thr Glu Arg Leu
            20                  25                  30

Tyr Ala Tyr His Leu Ser Arg Ala Ala Trp Tyr Gly Gly Leu Ala Val
        35                  40                  45

Leu Leu Gln Thr Ser Pro Glu Ala Pro Tyr Ile Tyr Ala Leu Leu Ser
    50                  55                  60

Arg Leu Phe Arg Ala Gln Asp Pro Asp Gln Leu Arg Gln His Ala Leu
65                  70                  75                  80

Ala Glu Gly Leu Thr Glu Glu Glu Tyr Gln Ala Phe Leu Val Tyr Ala
                85                  90                  95

Ala Gly Val Tyr Ser Asn Met Gly Asn Tyr Lys Ser Phe Gly Asp Thr
            100                 105                 110

Lys Phe Val Pro Asn Leu Pro Lys Glu Lys Leu Glu Arg Val Ile Leu
        115                 120                 125

Gly Ser Glu Ala Ala Gln Gln His Pro Glu Glu Val Arg Gly Leu Trp
    130                 135                 140

Gln Thr Cys Gly Glu Leu Met Phe Ser Leu Glu Pro Arg Leu Arg His
145                 150                 155                 160

Leu Gly Leu Gly Lys Glu Gly Ile Thr Thr Tyr Phe Ser Gly Asn Cys
                165                 170                 175

Thr Met Glu Asp Ala Lys Leu Ala Gln Asp Phe Leu Asp Ser Gln Asn
        180                 185                 190

Leu Ser Ala Tyr Asn Thr Arg Leu Phe Lys Glu Val Asp Gly Glu Gly
    195                 200                 205

Lys Pro Tyr Tyr Glu Val Arg Leu Ala Ser Val Leu Gly Ser Glu Pro
    210                 215                 220

Ser Leu Asp Ser Glu Val Thr Ser Lys Leu Lys Ser Tyr Glu Phe Arg
225                 230                 235                 240

Gly Ser Pro Phe Gln Val Thr Arg Gly Asp Tyr Ala Pro Ile Leu Gln
                245                 250                 255

Lys Val Val Glu Gln Leu Glu Lys Ala Lys Ala Tyr Ala Ala Asn Ser
            260                 265                 270

His Gln Gly Gln Met Leu Ala Gln Tyr Ile Glu Ser Phe Thr Gln Gly
        275                 280                 285

Ser Ile Glu Ala His Lys Arg Gly Ser Arg Phe Trp Ile Gln Asp Lys
    290                 295                 300

Gly Pro Ile Val Glu Ser Tyr Ile Gly Phe Ile Glu Ser Tyr Arg Asp
305                 310                 315                 320

Pro Phe Gly Ser Arg Gly Glu Phe Glu Gly Phe Val Ala Val Val Asn
                325                 330                 335

Lys Ala Met Ser Ala Lys Phe Glu Arg Leu Val Ala Ser Ala Glu Gln
            340                 345                 350

Leu Leu Lys Glu Leu Pro Trp Pro Pro Thr Phe Glu Lys Asp Lys Phe
        355                 360                 365

Leu Thr Pro Asp Phe Thr Ser Leu Asp Val Leu Thr Phe Ala Gly Ser
    370                 375                 380

Gly Ile Pro Ala Gly Ile Asn Ile Pro Asn Tyr Asp Asp Leu Arg Gln
385                 390                 395                 400

Thr Glu Gly Phe Lys Asn Val Ser Leu Gly Asn Val Leu Ala Val Ala
                405                 410                 415

Tyr Ala Thr Gln Arg Glu Lys Leu Thr Phe Leu Glu Glu Asp Asp Lys
            420                 425                 430

Asp Leu Tyr Ile Leu Trp Lys Gly Pro Ser Phe Asp Val Gln Val Gly
        435                 440                 445

Leu His Glu Leu Leu Gly His Gly Ser Gly Lys Leu Phe Val Gln Asp
    450                 455                 460

Glu Lys Gly Ala Phe Asn Phe Asp Gln Glu Thr Val Ile Asn Pro Glu
465                 470                 475                 480

Thr Gly Glu Gln Ile Gln Ser Trp Tyr Arg Ser Gly Glu Thr Trp Asp
                485                 490                 495

Ser Lys Phe Ser Thr Ile Ala Ser Ser Tyr Glu Glu Cys Arg Ala Glu
            500                 505                 510

Ser Val Gly Leu Tyr Leu Cys Leu His Pro Gln Val Leu Glu Ile Phe
        515                 520                 525

Gly Phe Glu Gly Ala Asp Ala Glu Asp Val Ile Tyr Val Asn Trp Leu
    530                 535                 540

Asn Met Val Arg Ala Gly Leu Leu Ala Leu Glu Phe Tyr Thr Pro Glu
545                 550                 555                 560

Ala Phe Asn Trp Arg Gln Ala His Met Gln Ala Arg Phe Val Ile Leu
                565                 570                 575

Arg Val Leu Leu Glu Ala Gly Glu Gly Leu Val Thr Ile Thr Pro Thr
            580                 585                 590

Thr Gly Ser Asp Gly Arg Pro Asp Ala Arg Val Arg Leu Asp Arg Ser
        595                 600                 605

Lys Ile Arg Ser Val Gly Lys Pro Ala Leu Glu Arg Phe Leu Arg Arg
    610                 615                 620

Leu Gln Val Leu Lys Ser Thr Gly Asp Val Ala Gly Gly Arg Ala Leu
625                 630                 635                 640

Tyr Glu Gly Tyr Ala Thr Val Thr Asp Ala Pro Pro Glu Cys Phe Leu
                645                 650                 655

Thr Leu Arg Asp Thr Val Leu Leu Arg Lys Glu Ser Arg Lys Leu Ile
            660                 665                 670

Val Gln Pro Asn Thr Arg Leu Glu Gly Ser Asp Val Gln Leu Leu Glu
        675                 680                 685

Tyr Glu Ala Ser Ala Ala Gly Leu Ile Arg Ser Phe Ser Glu Arg Phe
    690                 695                 700

Pro Glu Asp Gly Pro Glu Leu Glu Glu Ile Leu Thr Gln Leu Ala Thr
705                 710                 715                 720

Ala Asp Ala Arg Phe Trp Lys Gly Pro Ser Glu Ala Pro Ser Gly Gln
                725                 730                 735

Ala

<210>  2
<211>  21
<212>  PRT
<213>  Homo sapiens

<400>  2

Cys Glu Thr Val Ile Asn Pro Glu Thr Gly Glu Gln Ile Gln Ser Trp
1               5                   10                  15

Tyr Arg Ser Gly Glu
            20

<210>  3
<211>  6
<212>  PRT
<213>  Homo sapiens

<400>  3

Ile Asn Pro Glu Thr Gly
1               5

<210>  4
<211>  4
<212>  PRT
<213>  Homo sapiens

<400>  4

Glu Thr Gly Glu
1

<210>  5
<211>  116
<212>  PRT
<213>  Mus musculus

<400>  5

Gln Val Thr Leu Lys Glu Ser Gly Pro Gly Ile Leu Gln Pro Ser Gln

1               5                   10                  15

Thr Leu Ser Leu Thr Cys Ser Phe Ser Gly Phe Ser Leu Ser Thr Ser
            20                  25                  30

Gly Met Ser Val Gly Trp Ile Arg Gln Pro Ser Gly Lys Gly Leu Glu
        35                  40                  45

Trp Leu Ala His Ile Trp Trp Asn Asp Asn Lys Ser Tyr Asn Pro Ala
    50                  55                  60

Leu Lys Ser Arg Leu Thr Ile Ser Arg Asp Thr Ser Asn Asn Gln Val
65                  70                  75                  80

Phe Leu Lys Ile Ala Ser Val Val Thr Ala Asp Thr Gly Thr Tyr Phe
                85                  90                  95

Cys Ala Arg Asn Tyr Ser Tyr Asp Tyr Trp Gly Gln Gly Thr Thr Leu
            100                 105                 110

Thr Val Ser Ser
        115

<210>  6
<211>  112
<212>  PRT
<213>  Mus musculus

<400>  6

Asp Val Val Val Thr Gln Thr Pro Leu Ser Leu Ser Val Ser Leu Gly
1               5                   10                  15

Asp Pro Ala Ser Ile Ser Cys Arg Ser Ser Arg Ser Leu Val His Ser
            20                  25                  30

Ile Gly Ser Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Trp Thr Phe Gly Gly Gly Thr Lys Leu Glu Ile Lys

100                 105                 110

<210>  7
<211>  10
<212>  PRT
<213>  Mus musculus

<400>  7

Gly Phe Ser Leu Ser Thr Ser Gly Met Ser
1               5                   10

<210>  8
<211>  7
<212>  PRT
<213>  Mus musculus

<400>  8

Ile Trp Trp Asn Asp Asn Lys
1               5

<210>  9
<211>  8
<212>  PRT
<213>  Mus musculus

<400>  9

Ala Arg Asn Tyr Ser Tyr Asp Tyr
1               5

<210>  10
<211>  11
<212>  PRT
<213>  Mus musculus

<400>  10

Arg Ser Leu Val His Ser Ile Gly Ser Thr Tyr
1               5                   10

<210>  11
<211>  9
<212>  PRT
<213>  Mus musculus

<400>  11

Ser Gln Ser Thr His Val Pro Trp Thr
1               5

<210>  12
<211>  469
<212>  PRT
<213>  Homo sapiens

<400>  12

Met Asp Pro Lys Gly Ser Leu Ser Trp Arg Ile Leu Leu Phe Leu Ser
1               5                   10                  15

Leu Ala Phe Glu Leu Ser Tyr Gly Gln Ile Thr Leu Lys Glu Ser Gly
            20                  25                  30

Pro Thr Leu Val Lys Pro Thr Gln Thr Leu Thr Leu Thr Cys Thr Phe
        35                  40                  45

Ser Gly Phe Ser Leu Ser Thr Ser Gly Met Ser Val Gly Trp Ile Arg
    50                  55                  60

Gln Pro Pro Gly Lys Ala Leu Glu Trp Leu Ala His Ile Trp Trp Asn
65                  70                  75                  80

Asp Asn Lys Ser Tyr Asn Pro Ala Leu Lys Ser Arg Leu Thr Ile Thr
                85                  90                  95

Arg Asp Thr Ser Lys Asn Gln Val Val Leu Thr Met Thr Asn Met Asp
            100                 105                 110

Pro Val Asp Thr Gly Thr Tyr Tyr Cys Ala Arg Asn Tyr Ser Tyr Asp
        115                 120                 125

Tyr Trp Gly Gln Gly Thr Leu Val Thr Val Ser Ser Ala Ser Thr Lys
    130                 135                 140

Gly Pro Ser Val Phe Pro Leu Ala Pro Ser Ser Lys Ser Thr Ser Gly
145                 150                 155                 160

Gly Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Ser Leu Gly Thr Gln Thr Tyr Ile Cys Asn
    210                 215                 220

Val Asn His Lys Pro Ser Asn Thr Lys Val Asp Lys Lys Val Glu Pro
225                 230                 235                 240

Lys Ser Cys Asp Lys Thr His Thr Cys Pro Pro Cys Pro Ala Pro Glu

                245                 250                 255

Leu Leu Gly Gly Pro Ser Val Phe Leu Phe Pro Pro Lys Pro Lys Asp
            260                 265                 270

Thr Leu Met Ile Ser Arg Thr Pro Glu Val Thr Cys Val Val Val Asp
        275                 280                 285

Val Ser His Glu Asp Pro Glu Val Lys Phe Asn Trp Tyr Val Asp Gly
    290                 295                 300

Val Glu Val His Asn Ala Lys Thr Lys Pro Arg Glu Glu Gln Tyr Asn
305                 310                 315                 320

Ser Thr Tyr Arg Val Val Ser Val Leu Thr Val Leu His Gln Asp Trp
                325                 330                 335

Leu Asn Gly Lys Glu Tyr Lys Cys Lys Val Ser Asn Lys Ala Leu Pro
            340                 345                 350

Ala Pro Ile Glu Lys Thr Ile Ser Lys Ala Lys Gly Gln Pro Arg Glu
        355                 360                 365

Pro Gln Val Tyr Thr Leu Pro Pro Ser Arg Asp Glu Leu Thr Lys Asn
    370                 375                 380

Gln Val Ser Leu Thr Cys Leu Val Lys Gly Phe Tyr Pro Ser Asp Ile
385                 390                 395                 400

Ala Val Glu Trp Glu Ser Asn Gly Gln Pro Glu Asn Asn Tyr Lys Thr
                405                 410                 415

Thr Pro Pro Val Leu Asp Ser Asp Gly Ser Phe Phe Leu Tyr Ser Lys
            420                 425                 430

Leu Thr Val Asp Lys Ser Arg Trp Gln Gln Gly Asn Val Phe Ser Cys
        435                 440                 445

Ser Val Met His Glu Ala Leu His Asn His Tyr Thr Gln Lys Ser Leu
    450                 455                 460

Ser Leu Ser Pro Gly
465

<210> 13
<211> 239
<212> PRT
<213> Homo sapiens

<400>  13

Met Glu Thr Asp Thr Leu Leu Leu Trp Val Leu Leu Leu Trp Val Pro
1               5                   10                  15

Gly Ser Thr Gly Asp Ile Val Met Thr Gln Thr Pro Leu Ser Leu Ser
            20                  25                  30

Val Thr Pro Gly Gln Pro Ala Ser Ile Ser Cys Lys Ser Ser Arg Ser
        35                  40                  45

Leu Val His Ser Ile Gly Ser Thr Tyr Leu Tyr Trp Tyr Leu Gln Lys
    50                  55                  60

Pro Gly Gln Ser Pro Gln Leu Leu Ile Tyr Lys Val Ser Asn Arg Phe
65                  70                  75                  80

Ser Gly Val Pro Asp Arg Phe Ser Gly Ser Gly Ser Gly Thr Asp Phe
                85                  90                  95

Thr Leu Lys Ile Ser Arg Val Glu Ala Glu Asp Val Gly Val Tyr Tyr
            100                 105                 110

Cys Ser Gln Ser Thr His Val Pro Trp Thr Phe Gly Gly Gly Thr Lys
        115                 120                 125

Val Glu Ile Lys Arg Thr Val Ala Ala Pro Ser Val Phe Ile Phe Pro
    130                 135                 140

Pro Ser Asp Glu Gln Leu Lys Ser Gly Thr Ala Ser Val Val Cys Leu
145                 150                 155                 160

Leu Asn Asn Phe Tyr Pro Arg Glu Ala Lys Val Gln Trp Lys Val Asp
                165                 170                 175

Asn Ala Leu Gln Ser Gly Asn Ser Gln Glu Ser Val Thr Glu Gln Asp
            180                 185                 190

Ser Lys Asp Ser Thr Tyr Ser Leu Ser Ser Thr Leu Thr Leu Ser Lys
        195                 200                 205

Ala Asp Tyr Glu Lys His Lys Val Tyr Ala Cys Glu Val Thr His Gln
    210                 215                 220

Gly Leu Ser Ser Pro Val Thr Lys Ser Phe Asn Arg Gly Glu Cys
225                 230                 235

## Description

### Field of the invention

Subject matter of the present invention is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus, the method comprising:
- determining the level of dipeptidyl peptidase 3 (DPP3) in a sample of bodily fluid of said patient,
- comparing said level of determined DPP3 to a pre-determined threshold, and
- correlating said level of determined DPP3 with the risk of life-threatening deterioration or an adverse event, or
- correlating said level of determined DPP3 with the severity, or
- correlating said level of determined DPP3 with the success of a therapy or intervention, or
- correlating said level of DPP3 with a certain therapy or intervention, or
- correlating said level of DPP3 with the management of said patient.

Subject matter of the present invention is an inhibitor of the activity of DPP3 for use in therapy or intervention in a patient infected with a coronavirus.

### Background

Dipeptidyl peptidase 3 - also known as Dipeptidyl aminopeptidase III, Dipeptidyl arylamidase III, Dipeptidyl peptidase III, Enkephalinase B or red cell angiotensinase; short name: DPP3, DPPIII - is a metallopeptidase that removes dipeptides from physiologically active peptides, such as enkephalins and angiotensins. DPP3 was first identified and its activity measured in extracts of purified bovine anterior pituitary by Ellis & Nuenke 1967. The enzyme, which is listed as EC 3.4.14.4, has a molecular mass of about 83 kDa and is highly conserved in procaryotes and eucaryotes (*Prajapati & Chauhan 2011*). The amino acid sequence of the human variant is depicted in SEQ ID NO 1. Dipeptidyl peptidase III is a mainly cytosolic peptidase which is ubiquitously expressed. Despite lacking a signal sequence, a few studies reported membranous activity (*Lee & Snyder 1982*).
DPP3 is a zinc-depending exo-peptidase belonging to the peptidase family M49. It has a broad substrate specificity for oligopeptides from three/ four to ten amino acids of various compositions and is also capable of cleaving after proline. DPP3 is known to hydrolyze dipeptides from the N-terminus of its substrates, including angiotensin II, III and IV; Leu- and Met-enkephalin; endomorphin 1 and 2. The metallopeptidase DPP3 has its activity optimum at pH 8.0-9.0 and can be activated by addition of divalent metal ions, such as Co²⁺ and Mg²⁺. Structural analysis of DPP3 revealed the catalytic motifs HELLGH (human DPP3 [hDPP3] 450-455) and EECRAE (hDPP3 507-512), as well as following amino acids, that are important for substrate binding and hydrolysis: Glu316, Tyr, 318, Asp366, Asn391, Asn394, His568, Arg572, Arg577, Lys666 and Arg669 (*Prajapati & Chauhan 2011; Kumar et al. 2016*; numbering refers to the sequence of human DPP3, see SEQ ID NO. 1). Considering all known amino acids or sequence regions that are involved in substrate binding and hydrolysis, the active site of human DPP3 can be defined as the area between amino acids 316 and 669.

The most prominent substrate of DPP3 is angiotensin II (Ang II), the main effector of the renin-angiotensin system (RAS). The RAS is activated in cardiovascular diseases (Dostal et al. 1997. J Mol Cell Cardiol;29:2893-902*;* Roks et al. 1997. Heart Vessels. Suppl 12:119-24), sepsis, and septic shock (Corrêa et al. 2015. Crit Care 2015; 19:98). Ang II, in particular, has been shown to modulate many cardiovascular functions including the control of blood pressure and cardiac remodeling.

Recently, two assays were generated, characterized, and validated to specifically detect DPP3 in human bodily fluids (e.g., blood, plasma, serum): a luminescence immunoassay (LIA) to detect DPP3 protein concentration and an enzyme capture activity assay (ECA) to detect specific DPP3 activity (Rehfeld et al. 2019. JALM 3(6): 943-953). A washing step removes all interfering substances before the actual detection of DPP3 activity is performed. Both methods are highly specific and allow the reproducible detection of DPP3 in blood samples.

Circulating DPP3 levels were shown to be increased in cardiogenic shock patients and were associated with an increased risk of short-term mortality and severe organ dysfunction (Deaniau et al. 2019. Eur J Heart Fail. in press). Moreover, DPP3 measured at inclusion discriminated cardiogenic shock patients who did develop refractory shock vs. non-refractory shock and a DPP3 concentration ≥ 59.1 ng/mL was associated with a greater risk of death (Takagi et al. Eur J Heart Fail. in press).
WO2017/182561 describes methods for determining the total amount or active DPP3 in a sample of a patient for the diagnosis of a disease related to necrotic processes. It further describes a method of treatment of necrosis-related diseases by antibodies directed to DPP3.

WO2019/081595 describes DPP3 binder directed to and binding to specific DPP3 epitopes and its use in the prevention or treatment of diseases that are associated with oxidative stress.

Procizumab, a humanized monoclonal IgG1 antibody specifically binding circulating DPP3, targets and modulates DPP3 activity, an essential regulator of cardiovascular function. Its mode of action is relevant in acute diseases that are associated with massive cell death and uncontrolled release of intracellular DPP3 into the bloodstream. Translocated DPP3 remains active in the circulation where it cleaves bioactive peptides in an uncontrolled manner. Procizumab is able to block circulating DPP3, inhibiting bioactive peptide degradation in the bloodstream. This blockade results in stabilization of cardiovascular and renal function and reduction of short-term mortality. As shown in the Example part, preclinical studies of Procizumab in animal models of cardiovascular failure showed impressive and instant efficacy. As an example, injection of Procizumab in rats with shock-induced cardiovascular failure led to an instant normalization of shortening. In several preclinical cardiovascular failure models, Procizumab has shown to improve all clinically relevant endpoints in vivo. It normalizes ejection fraction and kidney function and reduces mortality.

Coronaviruscoronaviruses are widespread in humans and several other vertebrates and cause respiratory, enteric, hepatic, and neuro logic diseases. Notably, the severe acute respiratory syndrome coronavirus (SARS-CoV) in 2003 and Middle East respiratory syndrome coronavirus (MERS-CoV) in 2012 have caused human epidemics. Comparison with the SARS-CoV shows several significant differences and similarities. Both MERS CoV and SARS-CoV have much higher case fatality rates (40% and 10%, respectively) (de Wit et al. 2016. SARS and MERS: recent insights into emerging coronaviruses. Nat Rev Microbiol 14(8):523-34*;* Zhou et al. 2020. A pneumonia outbreak associated with a new coronavirus of probable bat origin. Nature in press). Though the current SARS CoV-2 shares 79% of its genome with SARS-CoV, it appears to be much more transmissible. Both SARS-CoVs enter the cell via the angiotensin converting enzyme 2 (ACE2) receptor (Wan et al. 2020. Receptor recognition by novel coronavirus from Wuhan: An analysis based on decade-long structural studies of SARS. J Virol in press).
The SARS-CoV-2 first predominantly infects lower airways and binds to ACE2 on alveolar epithelial cells. Both viruses are potent inducers of inflammatory cytokines. The "cytokine storm" or "cytokine cascade" is the postulated mechanism for organ damage. The virus activates immune cells and induces the secretion of inflammatory cytokines and chemokines into pulmonary vascular endothelial cells.

The clinical spectrum of SARS-CoV-2 infection appears to be wide, encompassing asymptomatic infection, mild upper respiratory tract illness, and severe viral pneumonia with respiratory failure and even death, with many patients being hospitalised with pneumonia (Huang et al. 2020 Clinical features of patients infected with 2019 novel coronavirus in Wuhan, China. Lancet 395: 497-506*;* Wang et al. 2020 Clinical characteristics of 138 hospitalized patients with 2019 novel coronavirus-infected pneumonia in Wuhan, China. JAMA in press*;* Chen et al. 2020. Epidemiological and clinical characteristics of 99 cases of 2019 novel coronavirus pneumonia in Wuhan, China: a descriptive study. Lancet 395: 507-13).

Very recently, older age, elevated d-dimer levels, and high SOFA score were proposed to help clinicians to identify at an early stage those patients with COVID-19 who have poor prognosis (Zhou et al. 2020. Clinical course and risk factors for mortality of adult inpatients with COVID-19 in Wuhan, China: a retrospective cohort study. The Lancet, in press.)

It is the surprising finding of the present invention, that in patients with coronavirus infection the level of DPP3 in a bodily fluid sample is to be used as a method for (a) diagnosing or predicting the risk of life-threatening deterioration or an adverse event or (b) prognosing the severity or (c) predicting or monitoring the success of a therapy or intervention in a patient infected with a coronavirus. Moreover, subject matter of the present invention is an inhibitor of the activity of DPP3 for use in therapy or intervention in a patient infected with a coronavirus.

### Description of the invention

Subject matter of the present invention is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus, the method comprising:
- determining the level of dipeptidyl peptidase 3 (DPP3) in a sample of bodily fluid of said patient,
- comparing said level of determined DPP3 to a pre-determined threshold, and
- correlating said level of determined DPP3 with the risk of life-threatening deterioration or an adverse event, or
- correlating said level of determined DPP3 with the severity, or
- correlating said level of determined DPP3 with the success of a therapy or intervention, or
- correlating said level of DPP3 with a certain therapy or intervention, or
- correlating said level of DPP3 with the management of said patient.

Subject-matter of the present application is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus wherein said coronavirus is selected from the group comprising Sars-CoV-1, Sars-CoV-2, MERS-CoV, in particular Sars-CoV-2.

Subject-matter of the present application is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to the present invention, wherein said adverse event is selected from the group comprising death, organ dysfunction, and shock.

Subject-matter of the present application is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to the present invention, wherein said level of determined DPP3 is above a pre-determined threshold.

Subject-matter of the present application is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to the present invention, wherein said predetermined threshold of DPP3 in a sample of bodily fluid of said subject is between 20 and 120 ng/mL, more preferred between 30 and 80 ng/mL, even more preferred between 40 and 60 ng/mL, most preferred said threshold is 50 ng/mL.

Subject-matter of the present application is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to the present invention, wherein said patient has a SOFA score equal or greater than 3, preferably equal or greater than 7 or said patient has a quickSOFA score equal or greater than 1, preferably equal or greater than 2.

Subject-matter of the present application is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to the present invention, wherein said patient has a level of D-dimer equal or greater than 0.5 µg/ml, preferably equal or greater than 1.0 µg/ml.

Subject-matter of the present application is a method method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to the present invention, wherein the level of DPP3 is determined by contacting said sample of bodily fluid with a capture binder that binds specifically to DPP3.

Subject-matter of the present application is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to the present invention, wherein said determination comprises the use of a capture-binder that binds specifically to full-length DPP3 wherein said capture-binder may be selected from the group of antibody, antibody fragment or non-IgG scaffold.

Subject-matter of the present application is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to the present invention, wherein the amount of DPP3 protein and/or DPP3 activity is determined in a bodily fluid sample of said subject and wherein said determination comprises the use of a capture-binder that binds specifically to full-length DPP3 wherein said capture-binder is an antibody.

Subject-matter of the present application is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to the present invention, wherein the amount of DPP3 protein and/or DPP3 activity is determined in a bodily fluid sample of said subject and wherein said determination comprises the use of a capture-binder that binds specifically to full-length DPP3 wherein said capture-binder is immobilized on a surface.

Subject-matter of the present application is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to the present invention, wherein the amount of DPP3 protein and/or DPP3 activity is determined in a bodily fluid sample of said subject and wherein said separation step is a washing step that removes ingredients of the sample that are not bound to said capture-binder from the captured DPP3.

Subject-matter of the present application is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to the present invention, wherein the method for determining DPP3 activity in a bodily fluid sample of said subject comprises the steps:
- contacting said sample with a capture-binder that binds specifically to full-length DPP3,
- separating DPP3 bound to said capture binder,
- adding substrate of DPP3 to said separated DPP3,
- quantifying of said DPP3 activity by measuring and quantifying the conversion of a substrate of DPP3.

Subject-matter of the present application is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to the present invention, wherein the DPP3 activity is determined in a bodily fluid sample of said subject and wherein DPP3 substrate conversion is detected by a method selected from the group comprising: fluorescence of fluorogenic substrates (e.g. Arg-Arg-βNA, Arg-Arg-AMC), color change of chromogenic substrates, luminescence of substrates coupled to aminoluciferin, mass spectrometry, HPLC/ FPLC (reversed phase chromatography, size exclusion chromatography), thin layer chromatography, capillary zone electrophoresis, gel electrophoresis followed by activity staining (immobilized, active DPP3) or western blot (cleavage products).

Subject-matter of the present application is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to the present invention, wherein the DPP3 activity is determined in a bodily fluid sample of said subject and wherein said substrate may be selected from the group comprising: angiotensin II, III and IV, Leu-enkephalin, Met-enkephalin, endomorphin 1 and 2, valorphin, β-casomorphin, dynorphin, proctolin, ACTH and MSH, or di-peptides coupled to a fluorophore, a chromophore or aminoluciferin wherein the di-peptide is Arg-Arg.

Subject-matter of the present application is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to the present invention, wherein the DPP3 activity is determined in a bodily fluid sample of said subject and wherein said substrate may be selected from the group comprising: A di-peptide coupled to a fluorophore, a chromophore or aminoluciferin wherein the di-peptide is Arg-Arg.

Subject-matter of the present application is a method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to claims the present invention, wherein said patient is treated with an inhibitor of DPP3 activity.

Subject-matter of the present application is an inhibitor of the activity of DPP3 for use in therapy or intervention in a patient infected with a coronavirus.

Subject-matter of the present application is an inhibitor of the activity of DPP3 for use in therapy or intervention in a patient infected with a coronavirus according to the present invention wherein said coronavirus is selected from the group comprising SARS-CoV-1, SARS-CoV-2, MERS-CoV, in particular SARS-CoV-2.

Subject-matter of the present application is an inhibitor of the activity of DPP3 for use in therapy or intervention in a patient infected with a coronavirus according to the present invention wherein said patient has a level of DPP3 in a sample of bodily fluid of said subject that is above a predetermined threshold when determined by a method according to any of the present invention.

Subject-matter of the present application is an inhibitor of the activity of DPP3 for use in therapy or intervention in a patient infected with a coronavirus according to the present invention, wherein said patient has a SOFA score equal or greater than 3, preferably equal or greater than 7 or said patient has a quickSOFA score equal or greater than 1, preferably equal or greater than 2.

Subject-matter of the present application is an inhibitor of the activity of DPP3 for use in therapy or intervention in a patient infected with a coronavirus according to the present invention, wherein said patient has a level of D-dimer equal or greater than 0.5 µg/ml, preferably equal or greater than 1.0 µg/ml.

Subject-matter of the present application is an inhibitor of the activity of DPP3 for use in therapy or intervention in a patient infected with a coronavirus according to the present invention, wherein the inhibitor of the activity of DPP3 is selected from the group comprising anti-DPP3 antibody or anti-DPP3 antibody fragment or anti-DPP3 non-Ig scaffold.

Subject-matter of the present application is an inhibitor of the activity of DPP3 for use in therapy or intervention in a patient infected with a coronavirus according to the present invention, wherein said inhibitor is an anti-DPP3 antibody or anti-DPP3 antibody fragment or anti-DPP3 non-Ig scaffold that binds an epitope of at least 4 to 5 amino acids in length comprised in SEQ ID No. 1.

Subject-matter of the present application is an inhibitor of the activity of DPP3 for use in therapy or intervention in a patient infected with a coronavirus according to the present invention, wherein said inhibitor is an anti-DPP3 antibody or anti-DPP3 antibody fragment or anti-DPP3 non-Ig scaffold that binds an epitope of at least 4 to 5 amino acids in length comprised in SEQ ID No. 2.

Subject-matter of the present application is an inhibitor of the activity of DPP3 for use in therapy or intervention in a patient infected with a coronavirus according to the present invention, wherein said inhibitor is an anti-DPP3 antibody or anti-DPP3 antibody fragment or anti-DPP3 non-Ig scaffold that exhibits a minimum binding affinity to DPP3 of equal or less than 10⁻⁷ M.

Subject-matter of the present application is an inhibitor of the activity of DPP3 for use in therapy or intervention in a patient infected with a coronavirus according to the present invention, wherein said inhibitor is an anti-DPP3 antibody or anti-DPP3 antibody fragment or anti-DPP3 non-Ig scaffold and inhibits activity of DPP3 of at least 10%, or at least 50%, more preferred at least 60%, even more preferred more than 70 %, even more preferred more than 80 %, even more preferred more than 90 %, even more preferred more than 95 %.

Subject-matter of the present application is an inhibitor of the activity of DPP3 for use in therapy or intervention in a patient infected with a coronavirus according to the present invention, wherein said antibody is a monoclonal antibody or monoclonal antibody fragment.

Subject-matter of the present application is an inhibitor of the activity of DPP3 for use in therapy or intervention in a patient infected with a coronavirus according to the present invention, wherein the complementarity determining regions (CDR's) in the heavy chain comprises the sequences:
SEQ ID NO.: 7, SEQ ID NO.: 8 and/ or SEQ ID NO.: 9
and the complementarity determining regions (CDR's) in the light chain comprises the sequences:
SEQ ID NO.: 10, KVS and/or SEQ ID NO.: 11.

Subject-matter of the present application is an inhibitor of the activity of DPP3 for use in therapy or intervention in a patient infected with a coronavirus according to the present invention, wherein said monoclonal antibody or antibody fragment is a humanized monoclonal antibody or humanized monoclonal antibody fragment.

Subject-matter of the present application is an inhibitor of the activity of DPP3 for use in therapy or intervention in a patient infected with a coronavirus, wherein the heavy chain comprises the sequence:
SEQ ID NO.: 12
and wherein the light chain comprises the sequence:
SEQ ID NO.: 13.

In one embodiment of the invention either the level of DPP3 protein and/or the level of active DPP3 is determined and compared to a threshold level.

In a specific embodiment of the invention a threshold of DPP3 in a sample of bodily fluid of said patient is between 20 and 120 ng/mL, more preferred between 30 and 80 ng/mL, even more preferred between 40 and 60 ng/mL, most preferred said threshold is 50 ng/mL.

In a specific embodiment of the invention a threshold for the level of DPP3 is the 5fold median concentration, preferably the 4fold median concentration, more preferred the 3fold median concentration, most preferred the 2fold median concentration of a normal healthy population.

The level of DPP3 as the amount of DPP3 protein and/ or DPP3 activity in a sample of bodily fluid of said subject may be determined by different methods, e.g. immunoassays, activity assays, mass spectrometric methods etc.

DPP3 activity can be measured by detection of cleavage products of DPP3 specific substrates. Known peptide hormone substrates include Leu-enkephalin, Met-enkephalin, endomorphin 1 and 2, valorphin, β-casomorphin, dynorphin, proctolin, ACTH (Adrenocorticotropic hormone) and MSH (melanocyte-stimulating hormone*; Abramić et al. 2000, Baršun et al. 2007, Dhanda et al. 2008*)*.* The cleavage of mentioned peptide hormones as well as other untagged oligopeptides (e.g. Ala-Ala-Ala-Ala, *Dhanda et al. 2008*) can be monitored by detection of the respective cleavage products. Detection methods include, but are not limited to, HPLC analysis (e.g. *Lee & Snyder 1982*), mass spectrometry (e.g. *Abramić et al. 2000*), H1-NMR analysis (e.g. *Vandenberg et al. 1985*), capillary zone electrophoresis (CE; e.g. *Baršun et al. 2007*), thin layer chromatography (e.g. *Dhanda et al. 2008*) or reversed phase chromatography (e.g. *Mazocco et al. 2006*).

Detection of fluorescence due to hydrolysis of fluorogenic substrates by DPP3 is a standard procedure to monitor DPP3 activity. Those substrates are specific di- or tripeptides (Arg-Arg, Ala-Ala, Ala-Arg, Ala-Phe, Asp-Arg, Gly-Ala, Gly-Arg, Gly-Phe, Leu-Ala, Leu-Gly, Lys-Ala, Phe-Arg, Suc-Ala-Ala-Phe) coupled to a fluorophore. Fluorophores include but are not limited to β-naphtylamide (2-naphtylamide, βNA, 2NA), 4-methoxy-β-naphtylamide (4-methoxy-2-naphtylamide) and 7-amido-4-methylcoumarin (AMC, MCA; *Abramić et al. 2000, Ohkubo et al. 1999*). Cleavage of these fluorogenic substrates leads to the release of fluorescent β-naphtylamine or 7-amino-4-methylcoumarin respectively. In a liquid phase assay or an ECA substrate and DPP3 are incubated in for example a 96 well plate format and fluorescence is measured using a fluorescence detector (*Ellis* & *Nuenke 1967*). Additionally, DPP3 carrying samples can be immobilized and divided on a gel by electrophoresis, gels stained with fluorogenic substrate (e.g. Arg-Arg-βNA) and Fast Garnet GBC and fluorescent protein bands detected by a fluorescence reader *(Ohkubo et al. 1999*). The same peptides (Arg-Arg, Ala-Ala, Ala-Arg, Ala-Phe, Asp-Arg, Gly-Ala, Gly-Arg, Gly-Phe, Leu-Ala, Leu-Gly, Lys-Ala, Phe-Arg, Suc-Ala-Ala-Phe) can be coupled to chromophores, such as p-nitroanilide diacetate. Detection of color change due to hydrolysis of chromogenic substrates can be used to monitor DPP3 activity.

Another option for the detection of DPP3 activity is a Protease-Glo^{™} Assay (commercially available at Promega). In this embodiment of said method DPP3 specific di- or tripeptides (Arg-Arg, Ala-Ala, Ala-Arg, Ala-Phe, Asp-Arg, Gly-Ala, Gly-Arg, Gly-Phe, Leu-Ala, Leu-Gly, Lys-Ala, Phe-Arg, Suc-Ala-Ala-Phe) are coupled to aminoluciferin. Upon cleavage by DPP3, aminoluciferin is released and serves as a substrate for a coupled luciferase reaction that emits detectable luminescence.

In a preferred embodiment DPP3 activity is measured by addition of the fluorogenic substrate Arg-Arg-βNA and monitoring fluorescence in real time.

In a specific embodiment of said method for determining active DPP3 in a bodily fluid sample of a subject said capture binder reactive with DPP3 is immobilized on a solid phase.

The test sample is passed over the immobile binder, and DPP3, if present, binds to the binder and is itself immobilized for detection. A substrate may then be added, and the reaction product may be detected to indicate the presence or amount of DPP3 in the test sample. For the purposes of the present description, the term "solid phase" may be used to include any material or vessel in which or on which the assay may be performed and includes, but is not limited to: porous materials, nonporous materials, test tubes, wells, slides, agarose resins (e.g. Sepharose from GE Healthcare Life Sciences), magnetic particals (e.g. Dynabeads^{™} or Pierce^{™} magnetic beads from Thermo Fisher Scientific), etc.

In another embodiment of the invention, the level of DPP3 is determined by contacting said sample of bodily fluid with a capture binder that binds specifically to DPP3.

In another preferred embodiment of the invention, said capture binder for determining the level of DPP3 may be selected from the group of antibody, antibody fragment or non-IgG scaffold.

In a specific embodiment of the invention, said capture binder is an antibody.

The amount of DPP3 protein and/ or DPP3 activity in a sample of bodily fluid of said subject may be determined for example by one of the following methods:

### 1. Luminescence immunoassay for the quantification of DPP3 protein concentrations (LIA) (Rehfeld et al., 2019 JALM 3(6): 943-953).

The LIA is a one-step chemiluminescence sandwich immunoassay that uses white high-binding polystyrene microtiter plates as solid phase. These plates are coated with monoclonal anti-DPP3 antibody AK2555 (capture antibody). The tracer anti-DPP3 antibody AK2553 is labeled with MA70-acridinium-NHS-ester and used at a concentration of 20 ng per well. Twenty microliters of samples (e.g. serum, heparin-plasma, citrate-plasma or EDTA-plasma derived from patients' blood) and calibrators are pipetted into coated white microtiter plates. After adding the tracer antibody AK2553, the microtiter plates are incubated for 3 h at room temperature and 600 rpm. Unbound tracer is then removed by 4 washing steps (350 µL per well). Remaining chemiluminescence is measured for Is per well by using a microtiter plate luminometer. The concentration of DPP3 is determined with a 6-point calibration curve. Calibrators and samples are preferably run in duplicate.

### 2. Enzyme capture activity assay for the quantification of DPP3 activity (ECA) (Rehfeld et al., 2019 JALM 3(6): 943-953).

The ECA is a DPP3-specific activity assay that uses black high-binding polystyrene microtiter plates as solid phase. These plates are coated with monoclonal anti-DPP3 antibody AK2555 (capture antibody). Twenty microliters of samples (e.g. serum, heparin-plasma, citrate-plasma, EDTA-plasma, cerebrospinal fluid and urine) and calibrators are pipetted into coated black microtiter plates. After adding assay buffer (200 µL), the microtiter plates are incubated for 2 h at 22°C and 600 rpm. DPP3 present in the samples is immobilized by binding to the capture antibody. Unbound sample components are removed by 4 washing steps (350 µL per well). The specific activity of immobilized DPP3 is measured by the addition of the fluorogenic substrate, Arg-Arg-β-Naphthylamide (Arg2-βNA), in reaction buffer followed by incubation at 37 °C for 1 h. DPP3 specifically cleaves Arg2-βNA into Arg-Arg dipeptide and fluorescent β-naphthylamine. Fluorescence is measured with a fluorometer using an excitation wavelength of 340 nm and emission is detected at 410 nm. The activity of DPP3 is determined with a 6-point calibration curve. Calibrators and samples are preferably run in duplicates.

### 3. Liquid-phase assay for the quantification of DPP3 activity (LAA) (modified from Jones et al., Analytical Biochemistry, 1982).

The LAA is a liquid phase assay that uses black non-binding polystyrene microtiter plates to measure DPP3 activity. Twenty microliter of samples (e.g. serum, heparin-plasma, citrate-plasma) and calibrators are pipetted into non-binding black microtiter plates. After addition of fluorogenic substrate, Arg2-βNA, in assay buffer (200 µL), the initial βNA fluorescence (T=0) is measured in a fluorimeter using an excitation wavelength of 340 nm and emission is detected at 410 nm. The plate is then incubated at 37 °C for 1 hour. The final fluorescence of (T=60) is measured. The difference between final and initial fluorescence is calculated. The activity of DPP3 is determined with a 6-point calibration curve. Calibrators and samples are preferably run in duplicates.

In a specific embodiment an assay is used for determining the level of DPP3, wherein the assay sensitivity of said assay is able to quantify the DPP3 of healthy subjects and is < 20 ng/ml, preferably < 30 ng/ml and more preferably < 40 ng/ml.

In a specific embodiment, said binder exhibits a binding affinity to DPP3 of at least 10⁷ M⁻¹, preferred 10⁸ M⁻¹, more preferred affinity is greater than 10⁹ M⁻¹, most preferred greater than 10¹⁰ M⁻¹. A person skilled in the art knows that it may be considered to compensate lower affinity by applying a higher dose of compounds and this measure would not lead out-of-the-scope of the invention.

In another embodiment of the invention, said sample of bodily fluid is selected from the group of whole blood, plasma, and serum.

A bodily fluid according to the present invention is in one particular embodiment a blood sample. A blood sample may be selected from the group comprising whole blood, serum and plasma. In a specific embodiment of the method said sample is selected from the group comprising human citrate plasma, heparin plasma and EDTA plasma.

To determine the affinity of the antibodies to DPP3 the kinetics of binding of DPP3 to immobilized antibody was determined by means of label-free surface plasmon resonance using a Biacore 2000 system (GE Healthcare Europe GmbH, Freiburg, Germany). Reversible immobilization of the antibodies was performed using an anti-mouse Fc antibody covalently coupled in high density to a CM5 sensor surface according to the manufacturer's instructions (mouse antibody capture kit; GE Healthcare), (Lorenz et al. 2011. Antimicrob Agents Chemother. 55 (1): 165-173).

In one embodiment such assay for determining the level of DPP3 is a sandwich immunoassay using any kind of detection technology including but not restricted to enzyme label, chemiluminescence label, electrochemiluminescence label, preferably a fully automated assay. In one embodiment of the diagnostic method such an assay is an enzyme labeled sandwich assay. Examples of automated or fully automated assay comprise assays that may be used for one of the following systems: Roche Elecsys^{®}, Abbott Architect^{®}, Siemens Centauer^{®}, Brahms Kryptor^{®}, BiomerieuxVidas^{®}, Alere Triage^{®}.

A variety of immunoassays are known and may be used for the assays and methods of the present invention, these include: mass spectrometry (MS), luminescence immunoassay (LIA), radioimmunoassays ("RIA"), homogeneous enzyme-multiplied immunoassays ("EMIT"), enzyme linked immunoadsorbent assays ("ELISA"), apoenzyme reactivation immunoassay ("ARIS"), luminescence-based bead arrays, magnetic beads based arrays, protein microarray assays, rapid test formats such as for instance dipstick immunoassays, immunochromatographic strip tests, rare cryptate assay and automated systems/ analysers.

In one embodiment of the invention it may be a so-called POC-test (point-of-care) that is a test technology, which allows performing the test within less than 1 hour near the patient without the requirement of a fully automated assay system. One example for this technology is the immunochromatographic test technology, e.g. a microfluidic device.

In a preferred embodiment said label is selected from the group comprising chemiluminescent label, enzyme label, fluorescence label, radioiodine label.

The assays can be homogenous or heterogeneous assays, competitive and non-competitive assays. In one embodiment, the assay is in the form of a sandwich assay, which is a non-competitive immunoassay, wherein the molecule to be detected and/or quantified is bound to a first antibody and to a second antibody. The first antibody may be bound to a solid phase, *e.g.* a bead, a surface of a well or other container, a chip or a strip, and the second antibody is an antibody which is labeled, *e.g.* with a dye, with a radioisotope, or a reactive or catalytically active moiety. The amount of labeled antibody bound to the analyte is then measured by an appropriate method. The general composition and procedures involved with "sandwich assays" are well-established and known to the skilled person (The Immunoassay Handbook, Ed. David Wild, Elsevier LTD, Oxford*;* 3rd ed. (May 2005), ISBN-13: 978-0080445267*;* Hultschig C et al., Curr Opin Chem Biol. 2006 Feb;10(1):4-10. PMID: 16376134).

In another embodiment the assay comprises two capture molecules, preferably antibodies which are both present as dispersions in a liquid reaction mixture, wherein a first labelling component is attached to the first capture molecule, wherein said first labelling component is part of a labelling system based on fluorescence- or chemiluminescence-quenching or amplification, and a second labelling component of said marking system is attached to the second capture molecule, so that upon binding of both capture molecules to the analyte a measurable signal is generated that allows for the detection of the formed sandwich complexes in the solution comprising the sample.

In another embodiment, said labeling system comprises rare earth cryptates or rare earth chelates in combination with fluorescence dye or chemiluminescence dye, in particular a dye of the cyanine type.

In the context of the present invention, fluorescence based assays comprise the use of dyes, which may for instance be selected from the group comprising FAM (5-or 6-carboxyfluorescein), VIC, NED, Fluorescein, Fluoresceinisothiocyanate (FITC), IRD-700/800, Cyanine dyes, auch as CY3, CY5, CY3.5, CY5.5, Cy7, Xanthen, 6-Carboxy-2',4',7',4,7-hexachlorofluorescein (HEX), TET, 6-Carboxy-4',5'-dichloro-2',7'-dimethodyfluorescein (JOE), N,N,N',N'-Tetramethyl-6-carboxyrhodamine (TAMRA), 6-Carboxy-X-rhodamine (ROX), 5-Carboxyrhodamine-6G (R6G5), 6-carboxyrhodamine-6G (RG6), Rhodamine, Rhodamine Green, Rhodamine Red, Rhodamine 110, BODIPY dyes, such as BODIPY TMR, Oregon Green, Coumarines such as Umbelliferone, Benzimides, such as Hoechst 33258; Phenanthridines, such as Texas Red, Yakima Yellow, Alexa Fluor, PET, Ethidiumbromide, Acridinium dyes, Carbazol dyes, Phenoxazine dyes, Porphyrine dyes, Polymethin dyes, and the like.

In the context of the present invention, chemiluminescence based assays comprise the use of dyes, based on the physical principles described for chemiluminescent materials in (Kirk-Othmer, Encyclopedia of chemical technology, 4th ed., executive editor, J. I. Kroschwitz; editor, M. Howe-Grant, John Wiley & Sons, 1993, vol. 15, p. 518-562*, incorporated herein by reference, including citations on pages 551-562*)*.* Preferred chemiluminescent dyes are acridiniumesters.

As mentioned herein, an "assay" or "diagnostic assay" can be of any type applied in the field of diagnostics. Such an assay may be based on the binding of an analyte to be detected to one or more capture probes with a certain affinity. Concerning the interaction between capture molecules and target molecules or molecules of interest, the affinity constant is preferably greater than 10⁸ M⁻¹.

In a specific embodiment at least one of said two binders is labeled in order to be detected.

The DPP3 levels of the present invention have been determined with the described DPP3-assays as outlined in the examples (Rehfeld et al. 2019. JALM 3(6): 943-953). The mentioned threshold values above might be different in other assays, if these have been calibrated differently from the assay systems used in the present invention. Therefore, the mentioned cut-off values above shall apply for such differently calibrated assays accordingly, taking into account the differences in calibration. One possibility of quantifying the difference in calibration is a method comparison analysis (correlation) of the assay in question with the respective biomarker assay used in the present invention by measuring the respective biomarker (e.g. DPP3) in samples using both methods. Another possibility is to determine with the assay in question, given this test has sufficient analytical sensitivity, the median biomarker level of a representative normal population, compare results with the median biomarker levels as described in the literature and recalculate the calibration based on the difference obtained by this comparison. With the calibration used in the present invention, samples from 5,400 normal (healthy) subjects (swedish single-center prospective population-based Study (MPP-RES)) have been measured: median (interquartile range) plasma DPP3 was 14.5 ng/ml (11.3 ng/ml - 19 ng/ml).

A particular advantage of the method of the present invention is that patients infected with a coronavirus can be stratified with respect to the required therapy, wherein said therapy is selected from the group comprising the administration of an inhibitor of DPP3 activity and an angiotensin-receptor-agonist and/ or a precursor thereof. The stratified patient groups may include patients that require an initiation of treatment and patients that do not require initiation of treatment.

Another particular advantage of the present invention is that the method can discriminate patients who are more likely to benefit from said therapy from patients who are not likely to benefit from said therapy.

In a preferred embodiment, the treatment with an inhibitor of DPP3 activity and/ or an angiotensin-receptor-agonist and/ or a precursor thereof is initiated or changed immediately upon provision of the result of the sample analysis indicating the level of DPP3 in the sample. In further embodiments, the treatment may be initiated within 12 hours, preferably 6, 4, 2, 1, 0.5, 0.25 hours or immediately after receiving the result of the sample analysis.

In some embodiments, the method comprises or consists of a single and/ or multiple measurement of DPP3 in a sample from a patient in a single sample and/or multiple samples obtained at essentially the same time point, in order to guide and/ or monitor and/ or stratify a therapy, wherein said therapy is the administration of an inhibitor of the activity of DPP3 and/ or an angiotensin-receptor-agonist and/ or a precursor thereof.

In one embodiment said angiotensin-receptor-agonist and/ or a precursor thereof is selected from the group comprising angiotensin I, angiotensin II, angiotensin III, angiotensin IV.

In a preferred embodiment the angiotensin II is angiotensin II acetate. Angiotensin II acetate is L-Aspartyl-L-arginyl-L-valyl-Ltyrosyl-L-isoleucyl-L-histidyl-L-prolyl-L-phenylalanine, acetate salt. The counter ion acetate is present in a non-stoichiometric ratio. The molecular formula of angiotensin II acetate is C₅₀H₇₁N₁₃O₁₂ • (C₂H₄O₂)n; (n=number of acetate molecules; theoretical n = 3) with an average molecular weight of 1046.2 (as free base).

The present invention further relates to a kit for carrying out the method of the invention, comprising detection reagents for determining the level of DPP3 in a sample from a patient.

It may also preferably be determined as a point of care assay that can be carried out directly at the place where the patient encounters the medical personnel, such as, for example, an emergency department or primary care unit. Furthermore, the assay for detection of may be an assay, preferably a duplex assay and/or a point of care assay that is automated or semi-automated.

In a preferred embodiment the present invention is related to methods and kits for determining the level of DPP3 and optionally further biomarkers in a sample from a patient.

Said further biomarkers may be selected from the group comprising D-Dimer, procalcitonin (PCT), C-reactive protein (CRP), lactate, bio-ADM, penKid, NT-proBNP, white blood cell count, lymphocyte count, neutrophil count, hemoglobin, platelet count, albumin, alanine transaminase, creatinine, blood urea, lactate dehydrogenase, creatinin kinase, cardiac troponin I, prothrombin time, serum ferritin, interleukin-6(IL-6), IL-10, IL-2, IL-7, tumor necrosis factor-α (TNF-α), granulocyte colony-stimulating factor (GCSF), IP-10, MCP-1, MIP-1α.

The present invention further relates to a kit for carrying out the method of the invention, comprising detection reagents for determining DPP3 in a sample from a patient, and reference data, such as a reference and/ or threshold level, corresponding to a level of DPP3 in said sample between 20 and 120 ng/mL, more preferred between 30 and 80 ng/mL, even more preferred between 40 and 60 ng/mL, most preferred 50 ng/mL, wherein said reference data is preferably stored on a computer readable medium and/or employed in the form of computer executable code configured for comparing the determined DPP3 to said reference data.

In one embodiment of the method described herein, the method additionally comprises comparing the determined level of DPP3 in patients infected with a coronavirus to a reference and/ or threshold level, wherein said comparing is carried out in a computer processor using computer executable code. The methods of the present invention may in part be computer-implemented. For example, the step of comparing the detected level of a marker, e.g. DPP3, with a reference and/ or threshold level can be performed in a computer system. For example, the determined values may be entered (either manually by a health professional or automatically from the device(s) in which the respective marker level(s) has/have been determined) into the computer-system. The computer-system can be directly at the point-of-care (e.g. primary care unit or ED) or it can be at a remote location connected via a computer network (e.g. via the internet, or specialized medical cloud-systems, optionally combinable with other IT-systems or platforms such as hospital information systems (HIS)). Alternatively, or in addition, the associated therapy guidance and/ or therapy stratification will be displayed and/or printed for the user (typically a health professional such as a physician).

In a specific embodiment of the invention said patient has been diagnosed with a coronavirus infection.

The term "coronavirus infection" is defined as an infection with coronavirus (Coronaviridae), a family of enveloped, positive-sense, single-stranded RNA viruses. The viral genome is 26-32 kilobases in length. The particles are typically decorated with large (∼20 nm), club- or petal-shaped surface projections (the "peplomers" or "spikes"), which in electron micrographs of spherical particles create an image reminiscent of the solar corona. Coronaviruses cause diseases in mammals and birds. In humans, the viruses cause respiratory infections, including the common cold, which are typically mild, though rarer forms such as SARS, MERS and COVID-19 can be lethal. The newest addition of human coronavirus strains is the SARS-CoV-2.

In a specific embodiment said infection with coronavirus is selected from the group comprising an infection with SARS-CoV-1, SARS-CoV-2, MERS-CoV, in particular SARS-CoV-2.

According to the WHO, severe acute respiratory infection (SARI) is currently defined as an acute respiratory infection (ARI) with history of fever or measured temperature ≥38 C° and cough, onset within the last -10 days, and requiring hospitalization. However, the absence of fever does not exclude viral infection.

SARS-CoV infection may present with mild, moderate, or severe illness; the latter includes severe pneumonia, acute respiratory distress syndrome (ARDS), sepsis and septic shock. Early identification of those with severe manifestations (see Table 1) allows for immediate optimized supportive care treatments and safe, rapid admission (or referral) to intensive care unit according to institutional or national protocols. For those with mild illness, hospitalization may not be required unless there is concern for rapid deterioration. All patients discharged home should be instructed to return to hospital if they develop any worsening of illness.

**Table 1. Clinical syndromes associated with 2019-nCoV infection (according to WHO guidance)**

| | |
|---|---|
| Uncomplicated illness | Patients with uncomplicated upper respiratory tract viral infection, may have non-specific symptoms such as fever, cough, sore throat, nasal congestion, malaise, headache, muscle pain or malaise. The elderly and immunosuppressed may present with atypical symptoms. These patients do not have any signs of dehydration, sepsis or shortness of breath. |
| Mild pneumonia | Patient with pneumonia and no signs of severe pneumonia. Child with non-severe pneumonia has cough or difficulty breathing + fast breathing: fast breathing (in breaths/min): <2 months, ≥60; 2-11 months, ≥50; 1-5 years, ≥40 and no signs of severe pneumonia. |
| Severe pneumonia | Adolescent or adult: fever or suspected respiratory infection, plus one of respiratory rate >30 breaths/min, severe respiratory distress, or SpO2 <90% on room air (adapted from [1]). Child with cough or difficulty in breathing, plus at least one of the following: central cyanosis or SpO2 <90%; severe respiratory distress (e.g. grunting, very severe chest indrawing); signs of pneumonia with a general danger sign: inability to breastfeed or drink, lethargy or unconsciousness, or convulsions. Other signs of pneumonia may be present: chest indrawing, fast breathing (in breaths/min): <2 months, ≥60; 2-11 months, ≥50; 1-5 years, ≥40.2 The diagnosis is clinical; chest imaging can exclude complications. |
| Acute Respiratory Distress Syndrome (ARDS) | Onset: new or worsening respiratory symptoms within one week of known clinical insult. Chest imaging (radiograph, CT scan, or lung ultrasound): bilateral opacities, not fully explained by effusions, lobar or lung collapse, or nodules. Origin of oedema: respiratory failure not fully explained by cardiac failure or fluid overload. Need objective assessment (e.g. echocardiography) to exclude hydrostatic cause of oedema if no risk factor present. Oxygenation (adults): |
| | - Mild ARDS: 200 mmHg < PaO2/FiO2 ≤ 300 mmHg (with PEEP or CPAP ≥5 cmH2O,7 or non-ventilated8) |
| | - Moderate ARDS: 100 mmHg < PaO2/FiO2 ≤200 mmHg with PEEP ≥5 cmH2O,7 or non-ventilated8) |
| | - Severe ARDS: PaO2/FiO2 ≤ 100 mmHg with PEEP ≥5 cmH2O,7 or non-ventilated8) |
| | - When PaO2 is not available, SpO2/FiO2 ≤315 suggests ARDS (including in non-ventilated patients) |
| | Oxygenation (children; note OI = Oxygenation Index and OSI = Oxygenation Index using SpO2): |
| | - Bilevel NIV or CPAP ≥5 cmH2O via full face mask: PaO2/FiO2 ≤ 300 mmHg or SpO2/FiO2 ≤264 |
| | - Mild ARDS (invasively ventilated): 4 ≤ OI < 8 or 5 ≤ OSI < 7.5 |
| | - Moderate ARDS (invasively ventilated): 8 ≤ OI < 16 or 7.5 ≤ OSI < 12.3 |
| | - Severe ARDS (invasively ventilated): OI ≥ 16 or OSI ≥ 12.3 |
| Sepsis | Adults: life-threatening organ dysfunction caused by a dysregulated host response to suspected or proven infection, with organ dysfunction. Signs of organ dysfunction include: altered mental status, difficult or fast breathing, low oxygen saturation, reduced urine output, fast heart rate, weak pulse, cold extremities or low blood pressure, skin mottling, or laboratory evidence of coagulopathy, thrombocytopenia, acidosis, high lactate or hyperbilirubinemia. |
| | Children: suspected or proven infection and ≥2 SIRS criteria, of which one must be abnormal temperature or white blood cell count. |
| Septic shock | Adults: persisting hypotension despite volume resuscitation, requiring vasopressors to maintain MAP ≥65 mmHg and serum lactate level >2 mmol/L. |
| | Children (any hypotension (SBP <5th centile or >2 SD below normal for age) or 2-3 of the following: altered mental state; tachycardia or bradycardia (HR <90 bpm or >160 bpm in infants and HR <70 bpm or >150 bpm in children); prolonged capillary refill (>2 sec) or warm vasodilation with bounding pulses; tachypnea; mottled skin or petechial or purpuric rash; increased lactate; oliguria; hyperthermia or hypothermia. |

| | |
|---|---|
| Oxygenation Index; OSI, Oxygenation Index using SpO₂; PaO₂, partial pressure of oxygen; PEEP, positive end-expiratory pressure; SBP, systolic blood pressure; SD, standard deviation; SIRS, systemic inflammatory response syndrome; SpO₂, oxygen saturation. *If altitude is higher than 1000m, then correction factor should be calculated as follows: PaO₂/FiO_{2X} Barometric pressure/760. | |

Septic shock is a potentially fatal medical condition that occurs when sepsis, which is organ injury or damage in response to infection, leads to dangerously low blood pressure and abnormalities in cellular metabolism. The Third International Consensus Definitions for Sepsis and Septic Shock (Sepsis-3) defines septic shock as a subset of sepsis in which particularly profound circulatory, cellular, and metabolic abnormalities are associated with a greater risk of mortality than with sepsis alone. Patients with septic shock can be clinically identified by a vasopressor requirement to maintain a mean arterial pressure of 65 mm Hg or greater and serum lactate level greater than 2 mmol/L (>18 mg/dL) in the absence of hypovolemia. This combination is associated with hospital mortality rates greater than 40% (Singer et al. 2016. JAMA. 315 (8): 801-10*)*. The primary infection is most commonly caused by bacteria, but also may be by fungi, viruses or parasites. It may be located in any part of the body, but most commonly in the lungs, brain, urinary tract, skin or abdominal organs. It can cause multiple organ dysfunction syndrome (formerly known as multiple organ failure) and death. Frequently, people with septic shock are cared for in intensive care units. It most commonly affects children, immunocompromised individuals, and the elderly, as their immune systems cannot deal with infection as effectively as those of healthy adults. The mortality rate from septic shock is approximately 25-50%.

As used herein, organ dysfunction denotes a condition or a state of health where an organ does not perform its expected function. "Organ failure" denotes an organ dysfunction to such a degree that normal homeostasis cannot be maintained without external clinical intervention. Said organ failure may pertain an organ selected from the group comprising kidney, liver, heart, lung, nervous system. By contrast, organ function represents the expected function of the respective organ within physiologic ranges. The person skilled in the art is aware of the respective function of an organ during medical examination.

Organ dysfunction may be defined by the sequential organ failure assessment score (SOFA-Score) or the components thereof. The SOFA score, previously known as the sepsis-related organ failure assessment score (Singer et al. 2016. JAMA 315(8):801-10) is used to track a person's status during the stay in an intensive care unit (ICU) to determine the extent of a person's organ function or rate of failure. The score is based on six different scores, one each for the respiratory, cardiovascular, hepatic, coagulation, renal and neurological systems each scored from 0 to 4 with an increasing score reflecting worsening organ dysfunction. The criteria for assessment of the SOFA score are described for example in Lamden et al. (for review see Lambden et al. 2019. Critical Care 23:374). SOFA score may traditionally be calculated on admission to ICU and at each 24-h period that follows. In particular, said organ dysfunction is selected from the group comprising renal decline, cardiac dysfunction, liver dysfunction or respiratory tract dysfunction.

The quick SOFA score (quickSOFA or qSOFA) was introduced by the Sepsis-3 group in February 2016 as a simplified version of the SOFA score as an initial way to identify patients at high risk for poor outcome with an infection (Angus et al. 2016. Critical Care Medicine. 44 (3): e113-e121*).* The qSOFA simplifies the SOFA score drastically by only including its three clinical criteria and by including "any altered mentation" instead of requiring a GCS <15. qSOFA can easily and quickly be repeated serially on patients. The score ranges from 0 to 3 points. One point is given for: low blood pressure (SBP ≤100 mmHg), high respiratory rate ((≥ 22 breaths/min) and altered mentation (GCS ≤ 15). The presence of 2 or more qSOFA points near the onset of infection was associated with a greater risk of death or prolonged intensive care unit stay. These are outcomes that are more common in infected patients who may be septic than those with uncomplicated infection. Based upon these findings, the Third International Consensus Definitions for Sepsis recommends qSOFA as a simple prompt to identify infected patients outside the ICU who are likely to be septic (Seymour et al. 2016. JAMA 315(8):762-774).

A life-threatening deterioration is defined as a condition of a patient associated with a high risk of death that involves vital organ system failure including central nervous system failure, renal failure, hepatic failure, metabolic failure or respiratory failure.

An adverse event is defined as death, organ dysfunction or shock.

In the present invention, the term "prognosis" or "prognosing" denotes a prediction of how a subject's (e.g. a patient's) medical condition will progress. This may include an estimation of the chance of recovery or the chance of an adverse outcome for said subject.

The term "therapy monitoring" in the context of the present invention refers to the monitoring and/or adjustment of a therapeutic treatment of said patient, for example by obtaining feedback on the efficacy of the therapy.

As used herein, the term "therapy guidance" refers to application of certain therapies or medical interventions based on the value of one or more biomarkers and/or clinical parameter and/or clinical scores.

Said clinical parameter or clinical scores are selected from the group comprising history of hypotension, vasopressor requirement, intubation, mechanical ventilation, Horowitz index, SOFA score, quick SOFA score.

The term "therapy stratification" in particular relates to grouping or classifying patients into different groups, such as therapy groups that receive or do not receive therapeutic measures depending on their classification.

Said therapy or intervention may be selected from the group comprising drug therapy, non-invasive ventilation, mechanical ventilation or extracorporeal membrane oxygenation (ECMO).

Non-invasive ventilation is the use of breathing support administered through a face mask, nasal mask, or a helmet. Air, usually with added oxygen, is given through the mask under positive pressure.

Mechanical ventilation or assisted ventilation, is the medical term for artificial ventilation where mechanical means are used to assist or replace spontaneous breathing. This may involve a machine called a ventilator, or the breathing may be assisted manually by a suitably qualified professional, such as an anesthesiologist, respiratory therapist (RT), Registered Nurse, or paramedic, by compressing a bag valve mask device. Mechanical ventilation is termed "invasive" if it involves any instrument inside the trachea through the mouth, such as an endotracheal tube or the skin, such as a tracheostomy tube. Face or nasal masks are used for non-invasive ventilation in appropriately selected conscious patients.

Extracorporeal membrane oxygenation (ECMO), also known as extracorporeal life support (ECLS), is an extracorporeal technique of providing prolonged cardiac and respiratory support to persons whose heart and lungs are unable to provide an adequate amount of gas exchange or perfusion to sustain life. The technology for ECMO is largely derived from cardiopulmonary bypass, which provides shorter-term support with arrested native circulation. ECMO works by removing blood from the person's body and artificially removing carbon dioxide from, and adding oxygen to, the patient's red blood cells. Generally, it is used either post-cardiopulmonary bypass or in late-stage treatment of a person with profound heart and/or lung failure, although it is now seeing use as a treatment for cardiac arrest in certain centers, allowing treatment of the underlying cause of arrest while circulation and oxygenation are supported. ECMO is also used to support patients with the acute viral pneumonia associated with COVID-19 in cases where artificial ventilation is not sufficient to sustain blood oxygenation levels.

Said drug therapy may be selected from the group comprising antiviral drugs, immunoglobulin from cured patients with COVID-19 pneumonia, neutralizing monoclonal antibodies targeting coronaviruses, immunoenhancers, camostat mesylate, coronaviral protease inhibitors (e.g. chymotrypsin-like inhibitors, papain-like protease inhibitors), spike (S) protein-angiotensin-converting enzyme-2 (ACE2) blockers (e.g. chloroquine, hydroxychloroquine, emodin, promazine), inhibitor of DPP3 activity and angiotensin-receptor-agonist and/or a precursor thereof.

Said neutralizing monoclonal antibodies targeting SARS-CoV and MERS-CoV may be selected from the group as summarized in Shanmugaraj et al. (Shanmugaraj et al. 2020. Asian Pac J. allergy Immunol 38: 10-18).

Said antiviral drugs may be selected from the group comprising Lopinavir, Ritonavir, Remdesivir, Nafamostat, Ribavirin, Oseltamivir, Penciclovir, Acyclovir, Ganciclovir, Favipiravir, Nitazoxanide, Nelfinavir, arbidol.

Said immunoenhancers may be selected from the group comprising interferons, intravenous gammaglobulin, thymosin α-1, levamisole, non-immunosuppressive derivatives of cyclosporin-A.

In one embodiment said angiotensin-receptor-agonist and/ or a precursor thereof is selected from the group comprising angiotensin I, angiotensin II, angiotensin III, angiotensin IV.

In a specific embodiment said angiotensin-receptor-rgonist and/ or a precursor thereof is administered to said patient if said predetermined level of DPP3 is above a threshold.

The term "patient" as used herein refers to a living human or non-human organism that is receiving medical care or that should receive medical care due to a disease. This includes persons with no defined illness who are being investigated for signs of pathology. Thus, the methods and assays described herein are applicable to both, human and veterinary disease.
The term "patient management" in the context of the present invention refers to:
- the decision for admission to hospital or intensive care unit,
- the decision for relocation of the patient to a specialized hospital or a specialized hospital unit,
- the evaluation for an early discharge from the intensive care unit or hospital,
- the allocation of resources (*e.g.* physician and/or nursing staff, diagnostics, therapeutics),
- the decision on therapeutic treatment.

In one embodiment of the invention said patient is a critically ill patient having an infection with coronavirus at the time the sample of bodily fluid of said patient is taken.

Inhibitors are molecules that preferably significantly inhibit DPP3 activity. Those molecules can be peptides and small molecules, antibodies, antibody fragments or non-Ig scaffolds.

Significantly inhibiting means inhibiting the activity of DPP3 more than 60%, preferably more than 70%, more preferably more than 80 %, preferably more than 90 %, more preferably almost or actually 100% inhibition.

The activity of DPP3 can be inhibited unspecifically by different general protease inhibitors (e.g. PMSF, TPCK), sulfhydryl reagents (e.g. pHMB, DTNB) and metal chelators (EDTA, o-phenantroline) (Abramić et al. 2000. Biological Chemistry, 381: 1233-1243*;* EP 2949332).

DPP3 activity can be further inhibited specifically by different kinds of compounds: an endogenous DPP3-inhibitor is the peptide spinorphin. Several synthetic derivatives of spinorphin, e.g. tynorphin, have been produced and shown to inhibit DPP3 activity to varying extents (Yamamoto et al. 2000. Life sciences 62 (19): 1767-1773). Other published peptide inhibitors of DPP3 are propioxatin A and B (US 4804676) and propioxatin A analogues *(*Inaoka et al. 1988. J. Biochem 104 (5): 706-711).

DPP3 can also be inhibited by small molecules such as fluostatins and benzimidazol derivatives. Fluostatins A and B are antibiotics produced in *Streptomyces sp.* TA-3391 that are non-toxic and strongly inhibit DPP3 activity. So far, 20 different derivatives of benzimidazol have been synthesized and published (Agić et al. 2007. Bioorganic Chemistry 35 (2): 153-169*;* Rastija et al. 2015. Acta Chimica Slovenica 62: 867-878), of which the two compounds 1' and 4' show the strongest inhibitory effect (Agić et al. 2007. Bioorganic Chemistry 35 (2): 153-169). Several dipeptidyl hydroxamic acids have been shown to inhibit DPP3 activity as well (Cvitešić et al., 2016. J Enzyme Inhib Med Chem 31(sup2):40-45).

In a specific embodiment of the invention said inhibitor of DPP3 activity is an anti-DPP3 antibody or anti-DPP3 antibody fragment or anti-DPP3 non-Ig scaffold.

Throughout the specification the "antibodies", or "antibody fragments" or "non-Ig scaffolds" in accordance with the invention are capable to bind DPP3, and thus are directed against DPP3, and thus can be referred to as "anti-DPP3 antibodies", "anti-DPP3 antibody fragments", or "anti-DPP3 non-Ig scaffolds".

The term "antibody" generally comprises monoclonal and polyclonal antibodies and binding fragments thereof, in particular Fc-fragments as well as so called "single-chain-antibodies" (*Bird et al. 1988*)*,* chimeric, humanized, in particular CDR-grafted antibodies, and dia or tetrabodies (*Holliger et al. 1993*). Also comprised are immunoglobulin-like proteins that are selected through techniques including, for example, phage display to specifically bind to the molecule of interest contained in a sample. In this context the term "specific binding" refers to antibodies raised against the molecule of interest or a fragment thereof. An antibody is considered to be specific, if its affinity towards the molecule of interest or the aforementioned fragment thereof is at least preferably 50-fold higher, more preferably 100-fold higher, most preferably at least 1000-fold higher than towards other molecules comprised in a sample containing the molecule of interest. It is well known in the art how to make antibodies and to select antibodies with a given specificity.

In one embodiment of the invention the anti-DPP3 antibody or anti-DPP3 antibody fragment or anti-DPP3 non-Ig scaffold is monospecific.

Monospecific anti-DPP3 antibody or monospecific anti-DPP3 antibody fragment or monospecific anti-DPP3 non-Ig scaffold means that said antibody or antibody fragment or non-Ig scaffold binds to one specific region encompassing at least 5 amino acids within the target DPP3 (SEQ ID No. 1). Monospecific anti-DPP3 antibody or monospecific anti-DPP3 antibody fragment or monospecific anti-DPP3 non-Ig scaffold are anti-DPP3 antibodies or anti-DPP3 antibody fragments or anti-DPP3 non-Ig scaffolds that all have affinity for the same antigen. Monoclonal antibodies are monospecific, but monospecific antibodies may also be produced by other means than producing them from a common germ cell.

In a specific embodiment said anti-DPP3 antibody, anti-DPP3 antibody fragment or anti-DPP3 non-Ig scaffold is an inhibiting antibody, fragment or non-Ig scaffold. Said anti-DPP3 antibody, anti-DPP3 antibody fragment or anti-DPP3 non-Ig scaffold is inhibiting the activity of DPP3 more than 60%, preferably more than 70%, more preferably more than 80 %, preferably more than 90 %, more preferably almost or actually 100%.

An antibody or fragment according to the present invention is a protein including one or more polypeptides substantially encoded by immunoglobulin genes that specifically binds an antigen. The recognized immunoglobulin genes include the kappa, lambda, alpha (IgA), gamma (IgG₁, IgG₂, IgG₃, IgG₄), delta (IgD), epsilon (IgE) and mu (IgM) constant region genes, as well as the myriad immunoglobulin variable region genes. Full-length immunoglobulin light chains are generally about 25 Kd or 214 amino acids in length.

Full-length immunoglobulin heavy chains are generally about 50 Kd or 446 amino acid in length. Light chains are encoded by a variable region gene at the NH₂-terminus (about 110 amino acids in length) and a kappa or lambda constant region gene at the COOH-terminus. Heavy chains are similarly encoded by a variable region gene (about 116 amino acids in length) and one of the other constant region genes.

The basic structural unit of an antibody is generally a tetramer that consists of two identical pairs of immunoglobulin chains, each pair having one light and one heavy chain. In each pair, the light and heavy chain variable regions bind to an antigen, and the constant regions mediate effector functions. Immunoglobulins also exist in a variety of other forms including, for example, Fv, Fab, and (Fab')₂, as well as bifunctional hybrid antibodies and single chains (*e.g.*, Lanzavecchia et al. 1987. Eur. J. Immunol. 17:105*;* Huston et al. 1988. Proc. Natl. Acad. Sci. U.S.A., 85:5879-5883*;* Bird et al. 1988. Science 242:423-426*;* Hood et al. 1984, Immunology, Benjamin, N.Y., 2nd ed*.;* Hunkapiller and Hood 1986. Nature 323:15-16). An immunoglobulin light or heavy chain variable region includes a framework region interrupted by three hypervariable regions, also called complementarity determining regions (CDR's) (see, Sequences of Proteins of Immunological Interest, E. Kabat et al. 1983, U.S. Department of Health and Human Services). As noted above, the CDRs are primarily responsible for binding to an epitope of an antigen. An immune complex is an antibody, such as a monoclonal antibody, chimeric antibody, humanized antibody or human antibody, or functional antibody fragment, specifically bound to the antigen.

Chimeric antibodies are antibodies whose light and heavy chain genes have been constructed, typically by genetic engineering, from immunoglobulin variable and constant region genes belonging to different species. For example, the variable segments of the genes from a mouse monoclonal antibody can be joined to human constant segments, such as kappa and gamma 1 or gamma 3. In one example, a therapeutic chimeric antibody is thus a hybrid protein composed of the variable or antigen-binding domain from a mouse antibody and the constant or effector domain from a human antibody, although other mammalian species can be used, or the variable region can be produced by molecular techniques. Methods of making chimeric antibodies are well known in the art, *e.g.*, see U.S. Patent No. 5,807,715. A "humanized" immunoglobulin is an immunoglobulin including a human framework region and one or more CDRs from a non-human (such as a mouse, rat, or synthetic) immunoglobulin. The non-human immunoglobulin providing the CDRs is termed a "donor" and the human immunoglobulin providing the framework is termed an "acceptor." In one embodiment, all the CDRs are from the donor immunoglobulin in a humanized immunoglobulin. Constant regions need not be present, but if they are, they must be substantially identical to human immunoglobulin constant regions, *i.e.,* at least about 85-90%, such as about 95% or more identical. Hence, all parts of a humanized immunoglobulin, except possibly the CDRs, are substantially identical to corresponding parts of natural human immunoglobulin sequences. A "humanized antibody" is an antibody comprising a humanized light chain and a humanized heavy chain immunoglobulin. A humanized antibody binds to the same antigen as the donor antibody that provides the CDR's. The acceptor framework of a humanized immunoglobulin or antibody may have a limited number of substitutions by amino acids taken from the donor framework. Humanized or other monoclonal antibodies can have additional conservative amino acid substitutions, which have substantially no effect on antigen binding or other immunoglobulin functions. Exemplary conservative substitutions are those such as gly, ala; val, ile, leu; asp, glu; asn, gln; ser, thr; lys, arg; and phe, tyr. Humanized immunoglobulins can be constructed by means of genetic engineering (*e.g.*, see U.S. Patent No. 5,585,089). A human antibody is an antibody wherein the light and heavy chain genes are of human origin. Human antibodies can be generated using methods known in the art. Human antibodies can be produced by immortalizing a human B cell secreting the antibody of interest. Immortalization can be accomplished, for example, by EBV infection or by fusing a human B cell with a myeloma or hybridoma cell to produce a trioma cell. Human antibodies can also be produced by phage display methods (see, *e.g.* WO91/17271; WO92/001047; WO92/20791), or selected from a human combinatorial monoclonal antibody library (see the Morphosys website). Human antibodies can also be prepared by using transgenic animals carrying a human immunoglobulin gene (for example, see WO93/12227; WO 91/10741).

Thus, the anti-DPP3 antibody may have the formats known in the art. Examples are human antibodies, monoclonal antibodies, humanized antibodies, chimeric antibodies, CDR-grafted antibodies. In a preferred embodiment antibodies according to the present invention are recombinantly produced antibodies as *e.g.* IgG, a typical full-length immunoglobulin, or antibody fragments containing at least the F-variable domain of heavy and/or light chain as *e.g.* chemically coupled antibodies (fragment antigen binding) including but not limited to Fab-fragments including Fab minibodies, single chain Fab antibody, monovalent Fab antibody with epitope tags, *e.g.* Fab-V5Sx2; bivalent Fab (mini-antibody) dimerized with the CH3 domain; bivalent Fab or multivalent Fab, *e.g.* formed via multimerization with the aid of a heterologous domain, *e.g.* via dimerization of dHLX domains, *e.g.* Fab-dHLX-FSx2; F(ab')₂-fragments, scFv-fragments, multimerized multivalent or/and multi-specific scFv-fragments, bivalent and/or bispecific diabodies, BITE^{®} (bispecific T-cell engager), trifunctional antibodies, polyvalent antibodies, *e.g.* from a different class than G; single-domain antibodies, *e.g.* nanobodies derived from camelid or fish immunoglobulines and numerous others.

In a preferred embodiment the anti-DPP3 antibody format is selected from the group comprising Fv fragment, scFv fragment, Fab fragment, scFab fragment, F(ab)₂ fragment and scFv-Fc Fusion protein. In another preferred embodiment the antibody format is selected from the group comprising scFab fragment, Fab fragment, scFv fragment and bioavailability optimized conjugates thereof, such as PEGylated fragments. One of the most preferred formats is the scFab format.

Non-Ig scaffolds may be protein scaffolds and may be used as antibody mimics as they are capable to bind to ligands or antigens. Non-Ig scaffolds may be selected from the group comprising tetranectin-based non-Ig scaffolds (*e.g.* described in US 2010/0028995), fibronectin scaffolds (e.g. described in EP 1 266 025; lipocalin-based scaffolds (*e.g.* described in WO 2011/154420); ubiquitin scaffolds (e.g. described in WO 2011/073214), transferrin scaffolds (e.g. described in US 2004/0023334), protein A scaffolds (e.g. described in EP 2 231 860), ankyrin repeat based scaffolds (e.g. described in WO 2010/060748), microproteins preferably microproteins forming a cysteine knot) scaffolds (e.g. described in EP 2314308), Fyn SH3 domain based scaffolds (e.g. described in WO 2011/023685) EGFR-A-domain based scaffolds (e.g. described in WO 2005/040229) and Kunitz domain based scaffolds (e.g. described in EP 1 941 867).

In one embodiment of the invention anti-DPP3 antibodies according to the present invention may be produced as outlined in Example 1 by synthesizing fragments of DPP3 as antigens or full-length DPP3. Thereafter, binder to said fragments are identified using the below described methods or other methods as known in the art.

Humanization of murine antibodies may be conducted according to the following procedure:
For humanization of an antibody of murine origin the antibody sequence is analyzed for the structural interaction of framework regions (FR) with the complementary determining regions (CDR) and the antigen. Based on structural modelling an appropriate FR of human origin is selected and the murine CDR sequences are transplanted into the human FR. Variations in the amino acid sequence of the CDRs or FRs may be introduced to regain structural interactions, which were abolished by the species switch for the FR sequences. This recovery of structural interactions may be achieved by random approach using phage display libraries or via directed approach guided by molecular modelling (Alma-cro and Fransson 2008. Humanization of antibodies. Front Biosci. 2008 Jan 1;13:1619-33).

In another preferred embodiment, the anti-DPP3 antibody, anti-DPP3 antibody fragment, or anti-DPP3 non-Ig scaffold is a full-length antibody, antibody fragment, or non-Ig scaffold.

In a preferred embodiment the anti-DPP3 antibody or an anti-DPP3 antibody fragment or anti-DPP3 non-Ig scaffold is directed to and can bind to an epitope of preferably at least 4 or at least 5 amino acids in length contained in DPP3 (SEQ ID No. 1).

In a preferred embodiment the anti-DPP3 antibody or an anti-DPP3 antibody fragment or anti-DPP3 non-Ig scaffold is directed to and can bind to an epitope of preferably at least 4 or at least 5 amino acids in length, wherein said antibody, fragment or non-Ig scaffold is directed to and can bind to an epitope comprised in SEQ ID NO.: 2, and wherein the epitope is comprised in DPP3 as depicted in SEQ ID NO.: 1.

In a preferred embodiment the anti-DPP3 antibody or an anti-DPP3 antibody fragment or anti-DPP3 non-Ig scaffold is directed to and can bind to an epitope of preferably at least 4 or at least 5 amino acids in length wherein said antibody, fragment or non-Ig scaffold is directed to and can bind to an epitope comprised in SEQ ID NO.: 3, and wherein the epitope is comprised in DPP3 as depicted in SEQ ID NO.: 1.

In a preferred embodiment the anti-DPP3 antibody or an anti-DPP3 antibody fragment or anti-DPP3 non-Ig scaffold is directed to and can bind to an epitope of preferably at least 4 or at least 5 amino acids in length wherein said antibody, fragment or non-Ig scaffold is directed to and can bind to an epitope comprised in SEQ ID NO.: 4, and wherein the epitope is comprised in DPP3 as depicted in SEQ ID NO.: 1.

In one specific embodiment of the invention the anti-DPP3 antibody or anti-DPP3 antibody fragment or anti-DPP3 non-Ig scaffold is provided for use in therapy or intervention in a patient infected with a coronavirus, wherein said antibody or fragment or scaffold binds to a region of preferably at least 4, or at least 5 amino acids within the sequence of DPP3 SEQ ID No.: 1.

In a preferred embodiment of the present invention said anti-DPP3 antibody or anti-DPP3 antibody fragment or anti-DPP3 non-Ig scaffold binds to a region or epitope of DPP3 that is located in SEQ ID No. 2.

In another preferred embodiment of the present invention said anti-DPP3 antibody or anti-DPP3 antibody fragment or anti-DPP3 non-Ig scaffold binds to a region or epitope of DPP3 that is located in SEQ ID No. 3.

In another preferred embodiment of the present invention said anti-DPP3 antibody or anti-DPP3 antibody fragment or anti-DPP3 non-Ig scaffold binds to a region or epitope of DPP3 that is located in SEQ ID No. 4.

An epitope, also known as antigenic determinant, is the part of an antigen that is recognized by the immune system, specifically by antibodies. For example, the epitope is the specific piece of the antigen to which an antibody binds. The part of an antibody that binds to the epitope is called a paratope. The epitopes of protein antigens are divided into two categories, conformational epitopes and linear epitopes, based on their structure and interaction with the paratope. Conformational and linear epitopes interact with the paratope based on the 3-D conformation adopted by the epitope, which is determined by the surface features of the involved epitope residues and the shape or tertiary structure of other segments of the antigen. A conformational epitope is formed by the 3-D conformation adopted by the interaction of discontiguous amino acid residues. A linear or a sequential epitope is an epitope that is recognized by antibodies by its linear sequence of amino acids, or primary structure and is formed by the 3-D conformation adopted by the interaction of contiguous amino acid residues.

In a specific embodiment of the invention the antibody is a monoclonal antibody or a fragment thereof. In one embodiment of the invention the anti-DPP3 antibody or the anti-DPP3 antibody fragment is a human or humanized antibody or derived therefrom. In one specific embodiment one or more (murine) CDR's are grafted into a human antibody or antibody fragment.

Subject matter of the present invention in one aspect is a human or humanized CDR-grafted antibody or antibody fragment thereof that binds to DPP3, wherein the human or humanized CDR-grafted antibody or antibody fragment thereof comprises an antibody heavy chain (H chain) comprising:
GFSLSTSGMS (SEQ ID No.: 7),
IWWNDNK (SEQ ID No.: 8),
ARNYSYDY (SEQ ID No.: 9)
and/or further comprises an antibody light chain (L chain) comprising:
RSLVHSIGSTY (SEQ ID No.: 10),
KVS (not part of the sequencing listing),
SQSTHVPWT (SEQ ID No.: 11).

In one specific embodiment of the invention subject matter of the present invention is a human or humanized monoclonal antibody that binds to DPP3 or an antibody fragment thereof that binds to DPP3 wherein the heavy chain comprises at least one CDR selected from the group comprising:
GFSLSTSGMS (SEQ ID No.: 7),
IWWNDNK (SEQ ID No.: 8),
ARNYSYDY (SEQ ID No.: 9)
and wherein the light chain comprises at least one CDR selected from the group comprising:
RSLVHSIGSTY (SEQ ID No.: 10),
KVS (not part of the sequencing listing),
SQSTHVPWT (SEQ ID No.: 11).

The anti-DPP3 antibody or anti-DPP3 antibody fragment or anti-DPP3 non-Ig scaffold according to the present invention exhibits an affinity towards human DPP3 in such that affinity constant is greater than 10⁻⁷ M, preferred 10⁻⁸ M, preferred affinity is greater than 10⁻⁹ M, most preferred higher than 10⁻¹⁰ M. A person skilled in the art knows that it may be considered to compensate lower affinity by applying a higher dose of compounds and this measure would not lead out-of-the-scope of the invention. The affinity constants may be determined according to the method as described in Example 1.

Subject matter of the present invention is a monoclonal antibody or fragment that binds to ADM or an antibody fragment thereof for use in therapy or intervention in a patient infected with a coronavirus, wherein said antibody or fragment comprises the following sequence as a variable heavy chain: 
SEQ ID No.: 5
QVTLKESGPGILQPSQTLSLTCSFSGFSLSTSGMSVGWIRQPSGKGLEWLAHIWWNDN
KSYNPALKSRLTISRDTSNNQVFLKIASVVTADTGTYFCARNYSYDYWGQGTTLTVS
S
 and comprises the following sequence as a variable light chain:

SEQ ID No.: 6
DVVVTQTPLSLSVSLGDPASISCRSSRSLVHSIGSTYLHWYLQKPGQSPKLLIYKVSNR
FSGVPDRFSGSGSGTDFTLKISRVEAEDLGVYFCSQSTHVPWTFGGGTKLEIK

Subject matter of the present invention is a human or humanized monoclonal antibody or fragment that binds to ADM or an antibody fragment thereof for use in therapy or intervention in a patient infected with a coronavirus, wherein said antibody or fragment comprises the following sequence as a heavy chain: 
SEQ ID No.: 12
MDPKGSLSWRILLFLSLAFELSYGQITLKESGPTLVKPTQTLTLTCTFSGFSLSTSGMS
VGWIRQPPGKALEWLAHIWWNDNKSYNPALKSRLTITRDTSKNQVVLTMTNMDPV
DTGTYYCARNYSYDYWGQGTLVTVSSASTKGPSVFPLAPSSKSTSGGTAALGCLVK
DYFPEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHEALH
NHYTQKSLSLSPG
 and comprises the following sequence as a light chain:

SEQ ID No.: 13
METDTLLLWVLLLWVPGSTGDIVMTQTPLSLSVTPGQPASISCKSSRSLVHSIGSTYL
YWYLQKPGQSPQLLIYKVSNRFSGVPDRFSGSGSGTDFTLKISRVEAEDVGVYYCSQS
THVPWTFGGGTKVEIKRTVAAPSVFIFPPSDEQLKSGTASVVCLLNNFYPREAKVQW
KVDNALQSGNSQESVTEQDSKDSTYSLSSTLTLSKADYEKHKVYACEVTHQGLSSPV
TKSFNRGEC

In a specific embodiment of the invention the antibody comprises the following sequence as a heavy chain: SEQ ID NO: 12
or a sequence that is > 95% identical to it, preferably > 98%, preferably > 99% and comprises the following sequence as a light chain: SEQ ID NO: 13
or a sequence that is > 95% identical to it, preferably > 98%, preferably > 99%.

To assess the identity between two amino acid sequences, a pairwise alignment is performed. Identity defines the percentage of amino acids with a direct match in the alignment.

The term "pharmaceutical formulation" means a pharmaceutical ingredient in combination with at least one pharmaceutically acceptable excipient, which is in such form as to permit the biological activity of a pharmaceutical ingredient contained therein to be effective, and which contains no additional components which are unacceptably toxic to a subject to which the formulation would be administered. The term "pharmaceutical ingredient" means a therapeutic composition which can be optionally combined with pharmaceutically acceptable excipients to provide a pharmaceutical formulation or dosage form.

Subject matter of the present invention is a pharmaceutical formulation for use in therapy or intervention in a patient infected with a coronavirus comprising an antibody or fragment or scaffold according to the present invention.

Subject matter of the present invention is a pharmaceutical formulation for use in therapy or intervention in a patient infected with a coronavirus according to the present invention, wherein said pharmaceutical formulation is a solution, preferably a ready-to-use solution.

Subject matter of the present invention is a pharmaceutical formulation for use in therapy or intervention in a patient infected with a coronavirus according to the present invention, wherein said pharmaceutical formulation is in a freeze-dried state.

Subject matter of the present invention is a pharmaceutical formulation for use in therapy or intervention in a patient infected with a coronavirus according to the present invention, wherein said pharmaceutical formulation is administered intra-muscular.

Subject matter of the present invention is a pharmaceutical formulation for use in therapy or intervention in a patient infected with a coronavirus according to the present invention, wherein said pharmaceutical formulation is administered intra-vascular.

Subject matter of the present invention is a pharmaceutical formulation for use in therapy or intervention in a patient infected with a coronavirus according to the present invention, wherein said pharmaceutical formulation is administered via infusion.

Subject matter of the present invention is a pharmaceutical formulation for use in therapy or intervention in a patient infected with a coronavirus according to the present invention, wherein said pharmaceutical formulation is to be administered systemically.

With the above context, the following consecutively numbered embodiments provide further specific aspects of the invention:

### Embodiments

1. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus, the method comprising:
   - determining the level of dipeptidyl peptidase 3 (DPP3) in a sample of bodily fluid of said patient,
   - comparing said level of determined DPP3 to a pre-determined threshold, and
   - correlating said level of determined DPP3 with the risk of life-threatening deterioration or an adverse event, or
   - correlating said level of determined DPP3 with the severity, or
   - correlating said level of determined DPP3 with the success of a therapy or intervention.
   - correlating said level of DPP3 with a certain therapy or intervention, or
   - correlating said level of DPP3 with the management of said patient.
2. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiment 1, wherein said coronavirus is selected from the group comprising SARS-CoV-1, SARS-CoV-2, MERS-CoV, in particular SARS-CoV-2
3. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiment 1 or 2, wherein said adverse event is selected from the group comprising death, organ dysfunction, and shock.
4. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiments 1 to 3, wherein said level of determined DPP3 is above a pre-determined threshold.
5. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiments 1 to 4, wherein said predetermined threshold of DPP3 in a sample of bodily fluid of said subject is between 20 and 120 ng/mL, more preferred between 30 and 80 ng/mL, even more preferred between 40 and 60 ng/mL, most preferred said threshold is 50 ng/mL.
6. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiments 1 to 5, wherein said patient has a SOFA score equal or greater than 3, preferably equal or greater than 7 or said patient has a quickSOFA score equal or greater than 1, preferably equal or greater than 2.
7. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiments 1 to 6, wherein said patient has a level of D-dimer equal or greater than 0.5 µg/ml, preferably equal or greater than 1 µg/ml.
8. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiments 1 to 7, wherein the level of DPP3 is determined by contacting said sample of bodily fluid with a capture binder that binds specifically to DPP3.
9. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiments 1 to 8, wherein said determination comprises the use of a capture-binder that binds specifically to full-length DPP3 wherein said capture-binder may be selected from the group of antibody, antibody fragment or non-IgG scaffold.
10. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiments 1 to 9, wherein the amount of DPP3 protein and/or DPP3 activity is determined in a bodily fluid sample of said subject and wherein said determination comprises the use of a capture-binder that binds specifically to full-length DPP3 wherein said capture-binder is an antibody.
11. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiments 1 to 10, wherein the amount of DPP3 protein and/or DPP3 activity is determined in a bodily fluid sample of said subject and wherein said determination comprises the use of a capture-binder that binds specifically to full-length DPP3 wherein said capture-binder is immobilized on a surface.
12. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiments 1 to 11, wherein the amount of DPP3 protein and/or DPP3 activity is determined in a bodily fluid sample of said subject and wherein said separation step is a washing step that removes ingredients of the sample that are not bound to said capture-binder from the captured DPP3.
13. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiments 1 to 12, wherein the method for determining DPP3 activity in a bodily fluid sample of said subject comprises the steps:
   - contacting said sample with a capture-binder that binds specifically to full-length DPP3,
   - separating DPP3 bound to said capture binder,
   - adding substrate of DPP3 to said separated DPP3,
   - quantifying of said DPP3 activity by measuring and quantifying the conversion of a substrate of DPP3.
14. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiments 1 to 13, wherein the DPP3 activity is determined in a bodily fluid sample of said subject and wherein DPP3 substrate conversion is detected by a method selected from the group comprising: fluorescence of fluorogenic substrates (e.g. Arg-Arg-βNA, Arg-Arg-AMC), color change of chromogenic substrates, luminescence of substrates coupled to aminoluciferin, mass spectrometry, HPLC/ FPLC (reversed phase chromatography, size exclusion chromatography), thin layer chromatography, capillary zone electrophoresis, gel electrophoresis followed by activity staining (immobilized, active DPP3) or western blot (cleavage products).
15. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiments 1 to 14, wherein the DPP3 activity is determined in a bodily fluid sample of said subject and wherein said substrate may be selected from the group comprising: angiotensin II, III and IV, Leu-enkephalin, Met-enkephalin, endomorphin 1 and 2, valorphin, β-casomorphin, dynorphin, proctolin, ACTH and MSH, or di-peptides coupled to a fluorophore, a chromophore or aminoluciferin wherein the di-peptide is Arg-Arg.
16. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiments 1 to 15, wherein the DPP3 activity is determined in a bodily fluid sample of said subject and wherein said substrate may be selected from the group comprising: A di-peptide coupled to a fluorophore, a chromophore or aminoluciferin wherein the di-peptide is Arg-Arg.
17. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to embodiments 1 to 16, wherein said patient is treated with an inhibitor of DPP3 activity and/or an angiotensin-receptor-agonist and/ or a precursor of said angiotensin-receptor-agonist.
18. Inhibitor of the activity of DPP3 and/or an angiotensin-receptor-agonist and/ or a precursor of said angiotensin-receptor-agonist for use in therapy or intervention in a patient infected with a coronavirus.
19. Inhibitor of the activity of DPP3 and/or an angiotensin-receptor-agonist and/ or a precursor of said angiotensin-receptor-agonist for use in therapy or intervention in a patient infected with a coronavirus according to embodiment 18 wherein said coronavirus is selected from the group comprising Sars-CoV-1, Sars-CoV-2, MERS-CoV, in particular Sars-CoV-2.
20. Inhibitor of the activity of DPP3 and/or an angiotensin-receptor-agonist and/ or a precursor of said angiotensin-receptor-agonist for use in therapy or intervention in a patient infected with a coronavirus according to embodiment 18 or 19 wherein said patient has a level of DPP3 in a sample of bodily fluid of said subject that is above a predetermined threshold when determined by a method according to any of embodiments 1 - 17.
21. Inhibitor of the activity of DPP3 and/or an angiotensin-receptor-agonist and/ or a precursor of said angiotensin-receptor-agonist for use in therapy or intervention in a patient infected with a coronavirus according to embodiments 18 to 20, wherein said patient has a SOFA score equal or greater than 3, preferably equal or greater than 7 or said patient has a quickSOFA score equal or greater than 1, preferably equal or greater than 2.
22. Inhibitor of the activity of DPP3 and/or an angiotensin-receptor-agonist and/ or a precursor of said angiotensin-receptor-agonist for use in therapy or intervention in a patient infected with a coronavirus according to embodiments 18 to 21, wherein said patient has a level of D-dimer equal or greater than 0.5 µg/ml, preferably 1.0 µg/ml.
23. Inhibitor of the activity of DPP3 for use in therapy or intervention in a patient infected with a coronavirus according to embodiments 20 - 22, wherein the inhibitor of the activity of DPP3 is selected from the group comprising anti-DPP3 antibody or anti-DPP3 antibody fragment or anti-DPP3 non-Ig scaffold.
24. Inhibitor of the activity of DPP3 for use in therapy or intervention in a patient infected with a coronavirus according to embodiments 20 - 23, wherein said inhibitor is an anti-DPP3 antibody or anti-DPP3 antibody fragment or anti-DPP3 non-Ig scaffold that binds an epitope of at least 4 to 5 amino acids in length comprised in SEQ ID No. 1.
25. Inhibitor of the activity of DPP3 for use in therapy or intervention in a patient infected with a coronavirus according to embodiments 20 - 24, wherein said inhibitor is an anti-DPP3 antibody or anti-DPP3 antibody fragment or anti-DPP3 non-Ig scaffold that binds an epitope of at least 4 to 5 amino acids in length comprised in SEQ ID No. 2.
26. Inhibitor of the activity of DPP3 for use in therapy or intervention in a patient infected with a coronavirus according to embodiments 20 - 25, wherein said inhibitor is an anti-DPP3 antibody or anti-DPP3 antibody fragment or anti-DPP3 non-Ig scaffold that exhibits a minimum binding affinity to DPP3 of equal or less than 10⁻⁷ M.
27. Inhibitor of the activity of DPP3 for use in therapy or intervention in a patient infected with a coronavirus according to embodiments 20 - 26, wherein said inhibitor is an anti-DPP3 antibody or anti-DPP3 antibody fragment or anti-DPP3 non-Ig scaffold and inhibits activity of DPP3 of at least 10%, or at least 50%, more preferred at least 60%, even more preferred more than 70 %, even more preferred more than 80 %, even more preferred more than 90 %, even more preferred more than 95 %.
28. Inhibitor of the activity of DPP3 for use in therapy or intervention in a patient infected with a coronavirus according to embodiments 20 - 27, wherein said antibody is a monoclonal antibody or monoclonal antibody fragment.
29. Inhibitor of the activity of DPP3 for use in therapy or intervention in a patient infected with a coronavirus according to embodiment 28, wherein the complementarity determining regions (CDR's) in the heavy chain comprises the sequences:
   SEQ ID NO.: 7, SEQ ID NO.: 8 and/ or SEQ ID NO.: 9
   and the complementarity determining regions (CDR's) in the light chain comprises the sequences:
   SEQ ID NO.: 10, KVS and/or SEQ ID NO.: 11.
30. Inhibitor of the activity of DPP3 for use in therapy or intervention in a patient infected with a coronavirus according to embodiment 29, wherein said monoclonal antibody or antibody fragment is a humanized monoclonal antibody or humanized monoclonal antibody fragment.
31. Inhibitor of the activity of DPP3 for use in therapy or intervention in a patient infected with a coronavirus according to embodiment 30, wherein the heavy chain comprises the sequence:
   SEQ ID NO.: 12
   and wherein the light chain comprises the sequence:
   SEQ ID NO.: 13.
32. Angiotensin-receptor-agonist and/ or a precursor thereof for use in therapy or intervention in a patient infected with a coronavirus according to embodiments 17 to 22, wherein said Angiotensin-receptor-agonist and/ or a precursor thereof is selected from the group comprising angiotensin I, angiotensin II, angiotensin III, angiotensin IV.

### FIGURE DESCRIPTION

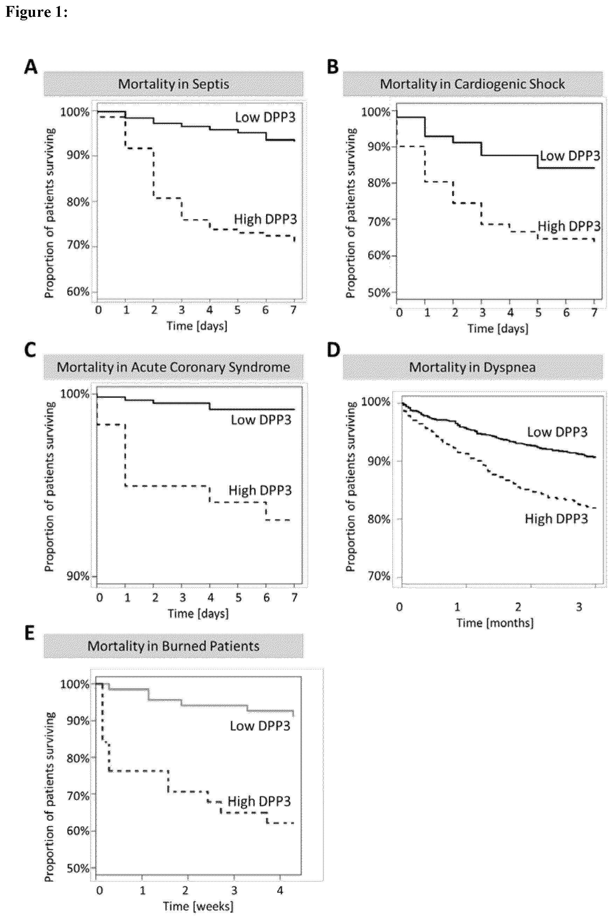

**Figure 1****:** Kaplan Meyer survival plots in relation to low (< 68.6 ng/mL) and high (≥ 68.6 ng/ml) DPP3 plasma concentrations
   **(A)** 7-Day survival of patients with sepsis/septic shock in relation to DPP3 plasma concentration (cut-off 68.6 ng/mL); **(B)** 7-Day survival of patients with cardiogenic shock in relation to DPP3 plasma concentration (cut-off 68.6 ng/mL); **(C)** 7-day survival of patients with acute myocardial infarction in relation to DPP3 plasma concentration (cut-off 68.6 ng/mL); **(D)** 3-month survival of patients with dyspnea in relation to DPP3 plasma concentration; **(E)** 4-week survival of burned patients in relation to DPP3 plasma concentration. **(F)** 7-Day survival of patients with septic shock in relation to DPP3 plasma concentration
**Figure 2****:** SDS-PAGE on a gradient gel (4-20%) of native hDPP3 purified from human erythrocyte lysate. Molecular weight marker is indicated as arrows.
**Figure 3****:** Experimental design - Effect of native DPP3 in an animal model.
**Figure 4****:** (A) DPP3 injection causes shortening fraction reduction and therefore leads to deteriorating heart function. (B) Decreased kidney function is also observed via increased renal resistive index.
**Figure 5****:** Association and dissociation curve of the AK1967-DPP3 binding analysis using Octet. AK1967 loaded biosensors were dipped into a dilution series of recombinant GST-tagged human DPP3 (100, 33.3, 11.1, 3.7 nM) and association and dissociation monitored.
**Figure 6****:** Western Blot of dilutions of blood cell lysate and detection of DPP3 with AK1967 as primary antibody.
**Figure 7****:** Inhibition curve of native DPP3 from blood cells with inhibitory antibody AK1967. Inhibition of DPP3 by a specific antibody is concentration dependent, with an IC₅₀ at ∼15 ng/ml when analyzed against 15 ng/ml DPP3.
**Figure 8****:** Experimental setup - Effect of Procizumab in sepsis-induced heart failure.
**Figure 9****:** Procizumab drastically improves shortening fraction (A) and mortality rate (B) in sepsis-induced heart failure rats.
**Figure 10****:** Experimental design - Isoproterenol-induced cardiac stress in mice followed by Procizumab treatment (B) and control (A).
**Figure 11****:** Procizumab improved shortening fraction (A) and reduced the renal resistive index (B) within 1 hour and 6 hours after administration, respectively, in isoproterenol-induced heart failure mice.
**Figure 12****:** Experimental setup - effect of Valsartan in healthy mice injected with DPP3.
**Figure 13****:** Reduction in the shortening fraction by DPP3 is rescued by the Valsartan treatment.
**Figure 14****:** High concentrations of DPP3 levels 24 hours after admission of septic patients were associated with worst SOFA scores.
**Figure 15****:** High cDPP3 plasma levels correlate with organ dysfunction in septic patients. Barplots of SOFA score in AdrenOSS-1 according to the evolution of DPP3 levels during ICU stay. HH: DPP3 above median on admission and at 24h; HL: above median on admission but below median at 24h; LL: below median on admission and at 24h; LH: below median on admission but above median at 24h.
**Figure 16****:** High concentrations of cDPP3 levels 24 hours after admission of septic patients were associated with worst SOFA scores by organ. (A) cardiac, (B) renal, (C) respiratory, (D) liver, (E) coagulation and (F) central nervous system SOFA scores values according to dynamics levels of cDPP3 between admission and 24h (HH: High/High, HL: High/Low, LH: Low/High, LL: Low/Low).

### EXAMPLES

### Example 1 - Methods for the measurement of DPP 3 protein and DPP3 activity

Generation of antibodies and determination DPP3 binding ability: Several murine antibodies were produced and screened by their ability of binding human DPP3 in a specific binding assay (see Table 2).

### Peptides/ conjugates for immunization:

DPP3 peptides for immunization were synthesized, see Table 2, (JPT Technologies, Berlin, Germany) with an additional N-terminal cystein (if no cystein is present within the selected DPP3-sequence) residue for conjugation of the peptides to Bovine Serum Albumin (BSA). The peptides were covalently linked to BSA by using Sulfolink-coupling gel (Perbio-science, Bonn, Germany). The coupling procedure was performed according to the manual of Perbio. Recombinant GST-hDPP3 was produced by USBio (United States Biological, Salem, MA, USA).

### Immunization of mice, immune cell fusion and screening:

Balb/c mice were intraperitoneally (i.p.) injected with 84 µg GST-hDPP3 or 100 µg DPP3-peptide-BSA-conjugates at day 0 (emulsified in TiterMax Gold Adjuvant), 84 µg or 100 µg at day 14 (emulsified in complete Freund's adjuvant) and 42 µg or 50 µg at day 21 and 28 (in incomplete Freund's adjuvant). At day 49 the animal received an intravenous (i.v.) injection of 42 µg GST-hDPP3 or 50 µg DPP3-peptide-BSA-conjugates dissolved in saline. Three days later the mice were sacrificed and the immune cell fusion was performed.

Splenocytes from the immunized mice and cells of the myeloma cell line SP2/0 were fused with 1 ml 50% polyethylene glycol for 30 s at 37°C. After washing, the cells were seeded in 96-well cell culture plates. Hybrid clones were selected by growing in HAT medium [RPMI 1640 culture medium supplemented with 20% fetal calf serum and HAT-Supplement]. After one week, the HAT medium was replaced with HT Medium for three passages followed by returning to the normal cell culture medium.

The cell culture supernatants were primarily screened for recombinant DPP3 binding IgG antibodies two weeks after fusion. Therefore, recombinant GST-tagged hDPP3 (USBiologicals, Salem, USA) was immobilized in 96-well plates (100 ng/ well) and incubated with 50 µl cell culture supernatant per well for 2 hours at room temperature. After washing of the plate, 50 µl / well POD-rabbit anti mouse IgG was added and incubated for 1 h at RT. After a next washing step, 50 µl of a chromogen solution (3,7 mM o-phenylen-diamine in citrate/ hydrogen phosphate buffer, 0.012% H₂O₂) were added to each well, incubated for 15 minutes at RT and the chromogenic reaction stopped by the addition of 50 µl 4N sulfuric acid. Absorption was detected at 490 mm.

The positive tested microcultures were transferred into 24-well plates for propagation. After retesting the selected cultures were cloned and re-cloned using the limiting-dilution technique and the isotypes were determined.

### Mouse monoclonal antibody production

Antibodies raised against GST-tagged human DPP3 or DPP3-peptides were produced via standard antibody production methods (*Marx et al. 1997*) and purified via Protein A. The antibody purities were ≥ 90% based on SDS gel electrophoresis analysis.

### Characterization of antibodies - binding to hDPP3 and/ or immunization peptide

To analyze the capability of DPP3/ immunization peptide binding by the different antibodies and antibody clones a binding assay was performed:

### a) Solid phase

Recombinant GST-tagged hDPP3 (SEQ ID NO. 1) or a DPP3 peptide (immunization peptide, SEQ ID NO. 2) was immobilized onto a high binding microtiter plate surface (96-Well polystyrene microplates, Greiner Bio-One international AG, Austria, 1 µg/well in coupling buffer [50 mM Tris, 100 mM NaCl, pH7,8], 1h at RT). After blocking with 5% bovine serum albumin, the microplates were vacuum dried.

### b) Labelling procedure (Tracer)

100 µg (100 µl) of the different antiDPP3 antibodies (detection antibody, 1 mg/ ml in PBS, pH 7.4) were mixed with 10 µl acridinium NHS-ester (1 mg/ml in acetonitrile, InVent GmbH, Germany; EP 0 353 971) and incubated for 30 min at room temperature. Labelled antiDPP3 antibody was purified by gel-filtration HPLC on Shodex Protein 5 µm KW-803 (Showa Denko, Japan). The purified labeled antibody was diluted in assay buffer (50 mmol/l potassium phosphate, 100 mmol/l NaCl, 10 mmol/l Na₂-EDTA, 5 g/l bovine serum albumin, 1 g/l murine IgG, 1 g/l bovine IgG, 50 µmol/l amastatin, 100 µmol/l leupeptin, pH 7.4). The final concentration was approx. 5-7^{∗}10⁶ relative light units (RLU) of labelled compound (approx. 20 ng labeled antibody) per 200 µl. acridinium ester chemiluminescence was measured by using a Centro LB 960 luminometer (Berthold Technologies GmbH & Co. KG).

### c) hDPP3 binding assay

The plates were filled with 200 µl of labelled and diluted detection antibody (tracer) and incubated for 2-4 h at 2-8 °C. Unbound tracer was removed by washing 4 times with 350 µl washing solution (20 mM PBS, pH 7.4, 0.1 % Triton X-100). Well-bound chemiluminescence was measured by using the Centro LB 960 luminometer (Berthold Technologies GmbH & Co. KG).

### Characterization of antibodies - hDPP3-inhibition analysis

To analyze the capability of DPP3 inhibition by the different antibodies and antibody clones a DPP3 activity assay with known procedure (Jones et al., 1982) was performed. Recombinant GST-tagged hDPP3 was diluted in assay buffer (25 ng/ ml GST-DPP3 in 50 mM Tris-HCl, pH7,5 and 100 µM ZnCl₂) and 200 µl of this solution incubated with 10 µg of the respective antibody at room temperature. After 1 hour of pre-incubation, fluorogenic substrate Arg-Arg-βNA (20 µl, 2mM) was added to the solution and the generation of free βNA over time was monitored using the Twinkle LB 970 microplate fluorometer (Berthold Technologies GmbH & Co. KG) at 37 °C. Fluorescence of βNA is detected by exciting at 340 nm and measuring emission at 410 nm. Slopes (in RFU/ min) of increasing fluorescence of the different samples are calculated. The slope of GST-hDPP3 with buffer control is appointed as 100 % activity. The inhibitory ability of a possible capture-binder is defined as the decrease of GST-hDPP3 activity by incubation with said capture-binder in percent.

The following table represents a selection of obtained antibodies and their binding rate in Relative Light Units (RLU) as well as their relative inhibitory ability (%; table 1). The monoclonal antibodies raised against the below depicted DPP3 regions, were selected by their ability to bind recombinant DPP3 and/ or immunization peptide, as well as by their inhibitory potential.

All antibodies raised against the GST-tagged, full length form of recombinant hDPP3 show a strong binding to immobilized GST-tagged hDPP3. Antibodies raised against the SEQ ID No.: 2 peptide bind to GST-hDPP3 as well. The SEQ ID No.: 2 antibodies also strongly bind to the immunization peptide.

**Table 2: list of antibodies raised against full-length or sequences of hDPP3 and their ability to bind hDPP3 (SEQ ID NO.: 1) or immunization peptide (SEQ ID NO.: 2) in RLU, as well as the maximum inhibition of recombinant GST-hDPP3.**

| **Sequence number** | **Antigene/Immunogen** | **hDPP3 region** | **Clone** | **hDPP3 binding [RLU]** | **immunization peptide binding [RLU]** | **Max inhibition of hDPP3** |
|---|---|---|---|---|---|---|
| **SEQ ID NO.: 1** | GST tagged recombinant FL-hDPP3 | 1-737 | 2552 | 3.053.621 | 0 | 65% |
| | | | 2553 | 3.777.985 | 0 | 35% |
| | | | 2554 | 1.733.815 | 0 | 30% |
| | | | 2555 | 3.805.363 | 0 | 25% |
| **SEQ ID NO.: 2** | CETVINPETGEQIQSWYRSGE | 474-493 | 1963 | 141.822 | 2.163.038 | 60% |
| | | | 1964 | 100.802 | 2.041.928 | 60% |
| | | | 1965 | 99.493 | 1.986.794 | 70% |
| | | | 1966 | 118.097 | 1.990.702 | 65% |
| | | | 1967 | 113.736 | 1.909.954 | 70% |
| | | | 1968 | 105.696 | 2.017.731 | 65% |
| | | | 1969 | 82.558 | 2.224.025 | 70% |

The development of a luminescence immunoassay for the quantification of DPP3 protein concentrations (DPP3-LIA) as well as an enzyme capture activity assay for the quantification of DPP3 activity (DPP3-ECA) have been described recently (Rehfeld et al. 2019. JALM 3(6): 943-953), which is incorporated here in its entirety by reference.

### Example 2 - DPP3 for prognosis of short-term mortality

DPP3 concentration in plasma of a variety of diseased patients was determined using a hDPP3 immunoassay (Rehfeld et al. 2019. JALM 3(6): 943-953) and related to the short term-mortality of the patients.

### Study Cohort - Sepsis and Septic Shock

Plasma samples form 574 patients from the Adrenomedullin and Outcome in Severe Sepsis and Septic Shock (AdrenOSS-1) study were screened for DPP3. AdrenOSS-1 is a prospective, observational, multinational study including 583 patients admitted to the intensive care unit with sepsis or septic shock (*Hollinger et al.. 2018*)*.* 292 patients were diagnosed with septic shock.

### Study Cohort - Cardiogenic Shock

Plasma samples from 108 patients that were diagnosed with cardiogenic shock were screened for DPP3. Blood was drawn within 6 h from detection of cardiogenic shock. Mortality was followed for 7 days.

### Study Cohort - Acute Coronary Syndrome

Plasma samples from 720 patients with acute coronary syndrome were screened for DPP3. Blood was drawn 24 hours after the onset of ChestPain. Mortality was followed for 7 days.

### Study Cohort - Dyspnea:

Plasma samples from 1440 patients presenting with dyspnea (shortness of breath) were collected immediately to their entry to the emergency department of Skåne University Hospital. Patients with dyspnea may suffer from acute coronary syndrome or congestive heart failure, beside others, and have a high risk for organ failure and short-term mortality. Mortality was followed for 3 months after presentation to the emergency department.

### Study Cohort - Burned Patients:

Plasma samples from 107 patients with severe burns (more than 15% of total body surface area) were screened for DPP3. Blood was drawn at admission to the hospital. Mortality was followed for 4 weeks.

### hDPP3 immunoassay:

An immune-assay (LIA) or an activity assays (ECA) detecting the amount of human DPP3 (LIA) or the activity of human DPP3 (ECA), respectively, was used for determining the DPP3 level in patient plasma. Antibody immobilization, labelling and incubation were performed as described in Rehfeld et al. (Rehfeld et al. 2019. JALM 3(6): 943-953).

### Results

Short-term patients' survival in Sepsis/Septic Shock was related to the DPP3 plasma concentration at admission. Patients with DPP3 plasma concentration above 68.6 ng/mL (3. Quartile) had an increased mortality risk compared to patients with DPP3 plasma concentrations below this threshold (Figure 1A). The same relation was visible when only the septic shock patients of this cohort were analyzed for their short-term outcome in relation to DPP3 plasma concentrations (Figure IF). Patients with an elevated DPP3 plasma concentration had an increased mortality risk compared to patients with a low DPP3 plasma concentration. When the same cut-off is applied to patients with cardiogenic shock, also an increased risk for short-term mortality within 7 days is observed in patients with high DPP3 (Figure 1B).

In addition, 28 day-survival of patients with acute coronary syndrome in relation to DPP3 is also increased when DPP3 is high and the respective cut-off of 68.6 ng/mL is applied (Figure 1C).

Applying this cut-off of 68.6 ng/mL to patients that suffer from Dyspnea, a significant increased mortality risk for patients with high DPP3 is detected within a follow-up of 3 months (Figure ID).

Furthermore, there was an increased risk for 4-week mortality in severely burned patients that have a high DPP3 concentration above the respective cut-off off 68.6 ng/mL (Figure IE).

### Example 3 - Purification of human native DPP3

Human erythrocyte lysate was applied on a total of 100 ml of Sepahrose 4B resin (Sigma-Aldrich) and the flow through was collected. The resin was washed with a total of 370 mL PBS buffer, pH 7.4 and the wash fraction was combined with the collected flow through, resulting in a total volume of 2370 mL.

For the immuno-affinity purification step, 110 mg of monoclonal anti-hDPP3 mAb AK2552 were coupled to 25.5 mL of UltraLink Hydrazide Resin (Thermo Fisher Scientific) according to the manufacturer's protocol (GlycoLink Immobilization Kit, Thermo Fisher Scientific). The coupling efficiency was 98%, determined by quantification of uncoupled antibody via Bradford-technique. The resin-antibody conjugate was equilibrated with 10 bed volumes of wash-binding buffer (PBS, 0.1% TritonX-100, pH 7.4), combined with 2370 mL of cleared red blood cell lysate and incubated at 4°C under continuous stirring for 2h. Consequently, 100 mL of the incubation mixture was spread on ten 15 mL polypropylene columns and the flow-through was collected by centrifugation at 1000xg for 30 seconds. This step was repeated several times resulting in 2.5 mL of DPP3-loaded resin per column. Each column was washed 5 times with 10 mL of wash-binding buffer using the gravity-glow approach. DPP3 was eluted by placing each column in 15-mL falcon tube containing 2 mL of neutralization buffer (1M Tris-HCl, pH 8.0), followed by addition of 10 mL of elution buffer (100 mM Glycine-HCl, 0.1% TritonX-100, pH 3.5) per column and immediate centrifugation for 30 seconds at 1000xg. The elution step was repeated 3 times in total resulting in 360 mL of combined eluates. The pH of the neutralized eluates was 8.0.

The combined eluates were loaded on a 5 mL HiTrap Q-sephare HP column (GE Healthcare) equilibrated with IEX-buffer A1 (100 mM Glycine, 150 mM Tris, pH 8.0) using the sample pump of the Äkta Start system (GE Healthcare). After sample loading, the column was washed with five column volumes of IEX Buffer A2 (12 mM NaH₂PO₄, pH 7.4) to remove unbound protein. Elution of DPP3 was achieved by applying a sodium chloride gradient over 10 column volumes (50 mL) in a range of 0 - 1 M NaCl using IEX-buffer B (12 mM NaH₂PO₄, 1 M NaCl, pH 7.4). The eluates were collected in 2 mL fractions. Buffers used for ion exchange chromatography were sterile filtered using a 0.22 µM bottle-top filter.

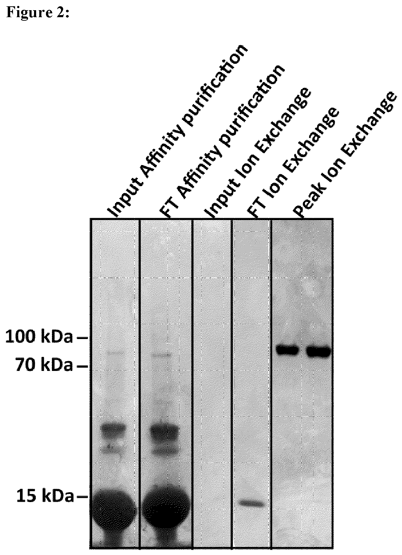

A purification table with the respective yields and activities of each purification step is given in table 3. Figure 2 shows an SDS-PAGE on a gradient gel (4-20%) of native hDPP3 purified from human erythrocyte lysate.

**Table 3: Purification of DPP3 from human erythrocytes**

| Step | DPP3 amount in % (LIA)^{a)} | Total protein in mg^{b)} | Total activity in µmol/min (ECA)^{c)} | Yield^{d)} in % | Specific activity in U/mg^{e)} | Purification factor^{f)} |
|---|---|---|---|---|---|---|
| Lysate | 100 | 204160 | 55 | 100 | 0.00027 | - |
| IAP | 80.6 | 71.2 | 46.1 | 84 | 0.65 | 2407 |
| IEX | 75 | 6.6 | 38.7 | 70 | 5.9 | 21852 |

| | | | | | | |
|---|---|---|---|---|---|---|
| ^{a)} Relative DPP3 amount was determined in all fractions using the DPP3-LIA assay. Amount of DPP3 in starting material was set to 100% and remaining DPP3 amount in purification fractions was correlated to the starting material. ^{b)} Total protein amount was determined using the method of Lowry modified by Peterson (Peterson 1977. Analytical Biochemistry 356:346-356). ^{c)} Total Arg₂-βNA hydrolyzing activity in µmol of substrate converted per minute was determined using the DPP3-ECA, calibrated via β-naphtylamine (0,05-100 µM). ^{d)} Purification yield was calculated form total Arg₂-βNA hydrolyzing activity. Arg₂-βNA hydrolyzing activity in starting material was set to 100%. ^{e)} Specific activity is defined as µmol of substrate converted per minute and mg of total protein. ^{f)} The purification factor is the quotient of specific activities after and before each purification step. | | | | | | |

### Example 4 - Effect of native DPP3 in an animal model

The effect of native hDPP3 injection in healthy mice was studied by monitoring the shortening fraction and renal resistive index.

Wild type Black 6 mice (8-12 weeks, group size refer to table 4) were acclimated during 2 weeks and a baseline echocardiography was done. The mice were randomly allocated to one of the two groups and, subsequently, native DPP3 protein or PBS were injected intravenously via a retro-orbital injection with a dose of 600 µg/kg for DPP3 protein.

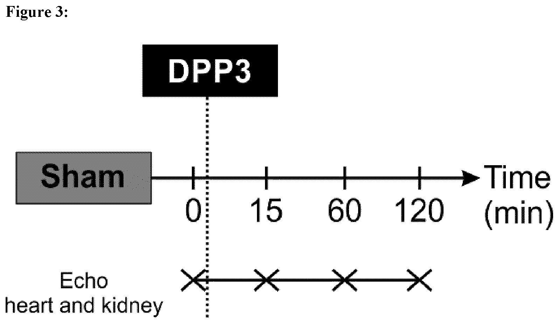

After DPP3 or PBS injection, cardiac function was assessed by echocardiography (*Gao et al. 2011)* and renal function assessed by renal resistive index (*Lubas et al., 2014, Dewitte et al, 2012)* at 15, 60 and 120 minutes (Figure 3).

**Table 4: list of experiment groups**

| **Group** | **Number of Animals** | **Treatment** |
|---|---|---|
| WT+PBS | 3 | PBS |
| WT+DPP3 | 4 | Native DPP3 |

### Results

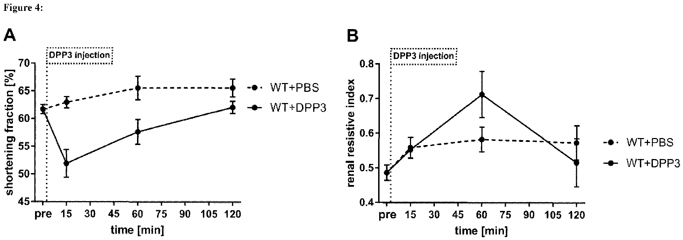

The mice treated with native DPP3 protein show significantly reduced shortening fraction compared to the control group injected with PBS (Fig 4A). The WT+DPP3 group also displays worsening renal function as observed by the renal resistive index increase (Figure 4B).

### Example 5 - Development of Procizumab

Antibodies raised against SEQ ID No.: 2 were characterized in more detail (epitope mapping, binding affinities, specificity, inhibitory potential). Here the results for clone 1967 of SEQ ID No.: 2 (AK1967; "Procizumab") are shown as an example.

### Determination of AK1967 epitope on DPP3:

For epitope mapping of AK1967 a number of N- or C-terminally biotinylated peptides were synthesized (peptides & elephants GmbH, Hennigsdorf, Germany). These peptides include the sequence of the full immunization peptide (SEQ ID No. 2) or fragments thereof, with stepwise removal of one amino acid from either C- or N-terminus (see table 6 for a complete list of peptides).

High binding 96 well plates were coated with 2 µg Avidin per well (Greiner Bio-One international AG, Austria) in coupling buffer (500 mM Tris-HCl, pH 7.8, 100 mM NaCl). Afterwards plates were washed and filled with specific solutions of biotinylated peptides (10 ng/ well; buffer - 1xPBS with 0.5% BSA)
Anti-DPP3 antibody AK1967 was labelled with a chemiluminescence label according to Example 1.

The plates were filled with 200 µl of labelled and diluted detection antibody (tracer) and incubated for 4 h at room temperature. Unbound tracer was removed by washing 4 times with 350 µl washing solution (20 mM PBS, pH 7.4, 0.1 % Triton X-100). Well-bound chemiluminescence was measured by using the Centro LB 960 luminometer (Berthold Technologies GmbH & Co. KG). Binding of AK1967 to the respective peptides is determined by evaluation of the relative light units (RLU). Any peptide that shows a significantly higher RLU signal than the unspecific binding of AK1967 is defined as AK1967 binder. The combinatorial analysis of binding and non-binding peptides reveals the specific DPP3 epitope of AK1967.

### Determination of binding affinities using Octet:

The experiment was performed using Octet Red96 (ForteBio). AK1967 was captured on kinetic grade anti-humanFc (AHC) biosensors. The loaded biosensors were then dipped into a dilution series of recombinant GST-tagged human DPP3 (100, 33.3, 11.1, 3.7 nM). Association was observed for 120 seconds followed by 180 seconds of dissociation. The buffers used for the experiment are depicted in table 5. Kinetic analysis was performed using a 1:1 binding model and global fitting.

**Table 5: Buffers used for Octet measurements**

| **Buffer** | **Composition** |
|---|---|
| Assay Buffer | PBS with 0.1% BSA, 0.02% Tween-21 |
| Regeneration Buffer | 10 mM Glycine buffer (pH 1.7) |
| Neutralization Buffer | PBS with 0.1% BSA, 0.02% Tween-21 |

### Western Blot analysis of Binding specificity of AK1967:

Blood cells from human EDTA-blood were washed (3x in PBS), diluted in PBS and lysed by repeated freeze-thaw-cycles. The blood cell lysate had a total protein concentration of 250 µg/ml, and a DPP3 concentration of 10 µg/ml. Dilutions of blood cell lysate (1:40, 1:80, 1:160 and 1:320) and of purified recombinant human His-DPP3 (31.25-500 ng/ml) were subjected to SDS-PAGE and Western Blot. The blots were incubated in 1.) blocking buffer (1xPBS-T with 5% skim milk powder), 2.) primary antibody solution (AK1967 1:2.000 in blocking buffer) and 3.) HRP labelled secondary antibody (goat anti mouse IgG, 1:1.000 in blocking buffer). Bound secondary antibody was detected using the Amersham ECL Western Blotting Detection Reagent and the Amersham Imager 600 UV (both from GE Healthcare).

### DPP3 inhibition assay:

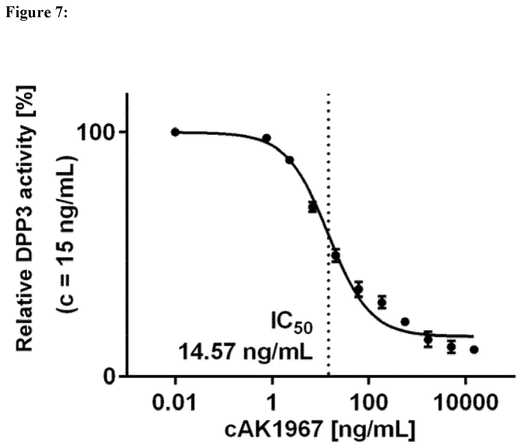

To analyze the capability of DPP3 inhibition by AK1967 a DPP3 activity assay with known procedure (*Jones et al., 1982*) was performed as described in example 1. The inhibitory ability AK1967 is defined as the decrease of GST-hDPP3 activity by incubation with said antibody in percent. The resulting lowered DPP3 activities are shown in an inhibition curve in Figure 7.

### Epitope mapping:

The analysis of peptides that AK1967 binds to and does not bind to revealed the DPP3 sequence INPETG (SEQ ID No.: 3) as necessary epitope for AK1967 binding (see table 6).

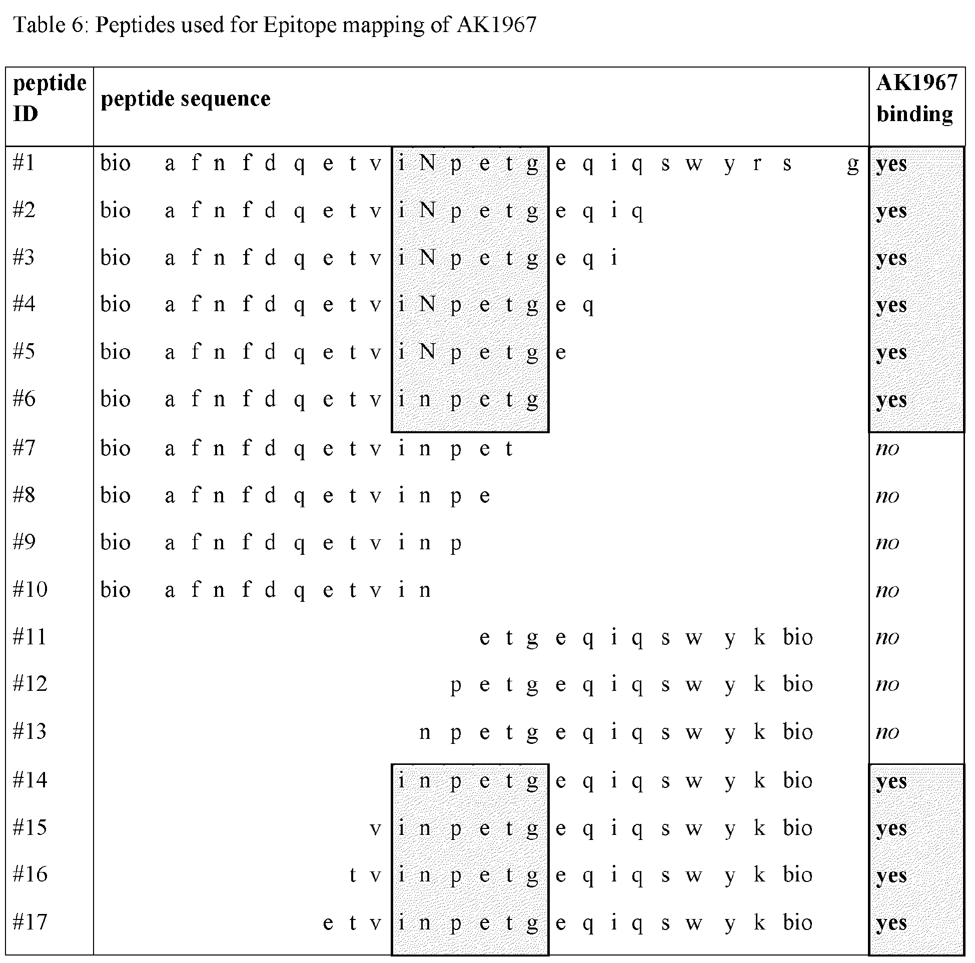

### Binding affinity:

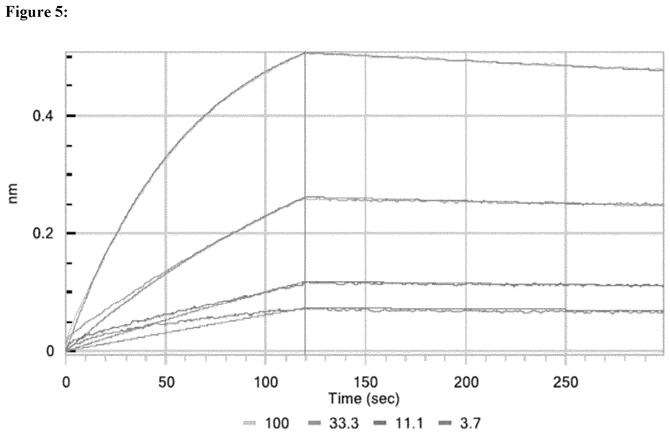

AK1967 binds with an affinity of 2.2^{∗}10⁻⁹ M to recombinant GST-hDPP3 (kinetic curves see Figure 5).

### Specificity and inhibitory potential:

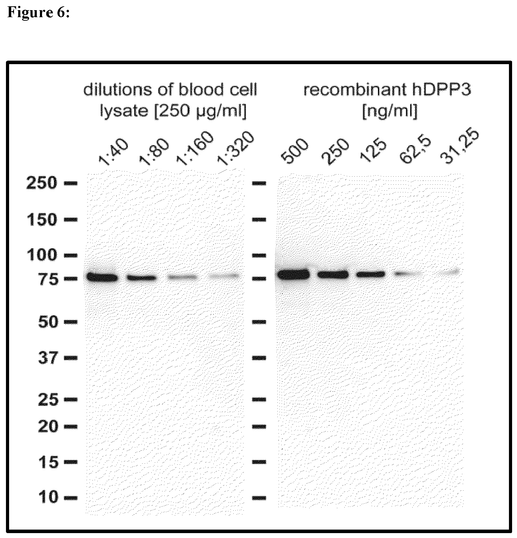

The only protein detected with AK1967 as primary antibody in lysate of blood cells was DPP3 at 80 kDa (Figure 6). The total protein concentration of the lysate was 250 µg/ml whereas the estimated DPP3 concentration is about 10 µg/ml. Even though there is 25 times more unspecific protein in the lysate, AK1967 binds and detects specifically DPP3 and no other unspecific binding takes place.

AK1967 inhibits 15 ng/ ml DPP3 in a specific DPP3 activity assay with an IC50 of about 15 ng/ml (Figure 7).

### Chimerization/ Humanization:

The monoclonal antibody AK1967 ("Procizumab"), with the ability of inhibiting DPP3 activity by 70 %, was chosen as possible therapeutic antibody and was also used as template for chimerization and humanization.

### Humanization of murine antibodies may be conducted according to the following procedure:

For humanization of an antibody of murine origin the antibody sequence is analyzed for the structural interaction of framework regions (FR) with the complementary determining regions (CDR) and the antigen. Based on structural modelling an appropriate FR of human origin is selected and the murine CDR sequences are transplanted into the human FR. Variations in the amino acid sequence of the CDRs or FRs may be introduced to regain structural interactions, which were abolished by the species switch for the FR sequences. This recovery of structural interactions may be achieved by random approach using phage display libraries or via directed approach guided by molecular modeling (Almagro and Fransson, 2008. Humanization of antibodies. Front Biosci. 13:1619-33*).*

With the above context, the variable region can be connected to any subclass of constant regions (IgG, IgM, IgE. IgA), or only scaffolds, Fab fragments, Fv, Fab and F(ab)2. In example 6 and 7 below, the murine antibody variant with an IgG2a backbone was used. For chimerization and humanization a human IgG1κ backbone was used.

For epitope binding only the Complementarity Determining Regions (CDRs) are of importance. The CDRs for the heavy chain and the light chain of the murine anti-DPP3 antibody (AK1967; "Procizumab") are shown in SEQ ID No. 7, SEQ ID No. 8 and SEQ ID No. 9 for the heavy chain and SEQ ID No. 10, sequence KVS and SEQ ID No. 11 for the light chain, respectively.

Sequencing of the anti-DPP3 antibody (AK1967; "Procizumab") revealed an antibody heavy chain variable region (H chain) according to SEQ ID No.: 12 and an antibody light chain variable region (L chain) according to SEQ ID No.: 13.

### Example 6 - Effect of Procizumab in sepsis-induced heart failure

In this experiment, the effect of Procizumab injection in sepsis-induced heart failure rats (*Rittirsch et al. 2009*) was studied by monitoring the shortening fraction.

### CLP model of septic shock:

Male Wistar rats (2-3 months, 300 to 400 g, group size refers to table 7) from the Centre d'élevage Janvier (France) were allocated randomly to one of three groups. All the animals were anesthetized using ketamine hydrochloride (90 mg/ kg) and xylazine (9 mg/ kg) intraperitoneally (i.p.). For induction of polymicrobial sepsis, cecal ligation and puncture (CLP) was performed using Rittirsch's protocol with minor modification. A ventral midline incision (1.5 cm) was made to allow exteriorization of the cecum. The cecum is then ligated just below the ileocecal valve and punctured once with an 18-gauge needle. The abdominal cavity is then closed in two layers, followed by fluid resuscitation (3 ml/ 100 g body of weight of saline injected subcutaneously) and returning the animal to its cage. Sham animals were subjected to surgery, without getting their cecum punctured. CLP animals were randomized between placebo and therapeutic antibody.

### Study design:

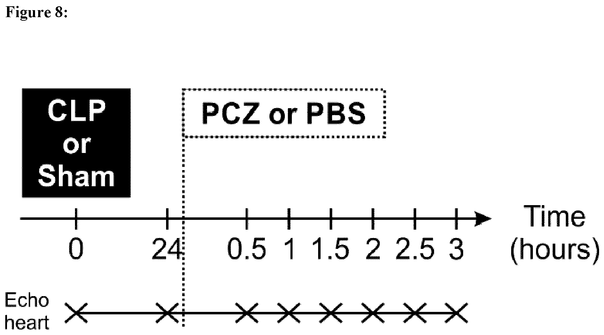

The study flow is depicted in Figure 8. After CLP or sham surgery the animals were allowed to rest for 20 hours with free access to water and food. Afterwards they were anesthetized, tracheotomy done and arterial and venous line laid. At 24 hours after CLP surgery either AK1967 or vehicle (saline) were administered with 5 mg/kg as a bolus injection followed by a 3h infusion with 7.5 mg/kg. As a safety measure, hemodynamics were monitored invasively and continuously from t = 0 till 3 h.

At t=0 (baseline) all CLP animals are in septic shock and developed a decrease in heart function (low blood pressure, low shortening fraction). At this time point Procizumab or vehicle (PBS) were injected (i.v.) and saline infusion was started. There were 1 control group and 2 CLP groups which are summarized in the table below (table 7). At the end of the experiment, the animals were euthanized, and organs harvested for subsequent analysis.

**Table 7: list of experimental groups**

| **Group** | **Number of Animals** | **CLP** | **Treatment** |
|---|---|---|---|
| Sham | 7 | No | PBS |
| CLP-PBS | 6 | Yes | PBS |
| CLP-PCZ | 4 | Yes | PCZ |

### Invasive Blood Pressure:

Hemodynamic variables were obtained using the AcqKnowledge system (BIOPAC Systems, Inc., USA). It provides a fully automated blood pressure analysis system. The catheter is connected to the BIOPAC system through a pressure sensor.

For the procedure, rats were anesthetized (ketamine and xylazine). Animals were moved to the heating pad for the desired body temperature to 37-37.5 °C. The temperature feedback probe was inserted into the rectum. The rats were placed on the operating table in a supine position. The trachea was opened and a catheter (16G) was inserted for an external ventilator without to damage carotid arteries and vagus nerves. The arterial catheter was inserted into the right carotid artery. The carotid artery is separate from vagus before ligation.

A central venous catheter was inserted through the left jugular vein allowing administration of PCZ or PBS.

Following surgery, the animals were allowed to rest for the stable condition prior to hemodynamic measurements. Then baseline blood pressure (BP) were recorded. During the data collection, saline infusion via arterial line was stopped.

### Echocardiography:

Animals were anesthetized using ketamine hydrochloride. Chests were shaved and rats were placed in decubitus position.

For transthoracic echocardiographic (TTE) examination a commercial GE Healthcare Vivid 7 Ultra-sound System equipped with a high frequency (14-MHz) linear probe and 10-MHz cardiac probe was used. All examinations were recorded digitally and stored for subsequent off-line analysis.

Grey scale images were recorded at a depth of 2 cm. Two-dimensional examinations were initiated in a parasternal long axis view to measure the aortic annulus diameter and the pulmonary artery diameter. M-mode was also employed to measure left ventricular (LV) dimensions and assess fractional shortening (FS%). LVFS was calculated as LV end-diastolic diameter - LV end-systolic diameter / LV end-diastolic diameter and expressed in %. The time of end-diastole was therefore defined at the maximal diameter of the LV. Accordingly, end-systole was defined as the minimal diameter in the same heart cycle. All parameters were measured manually. Three heart cycles were averaged for each measurement.

From the same parasternal long axis view, pulmonary artery flow was recorded using pulsed wave Doppler. Velocity time integral of pulmonary artery outflow was measured.

From an apical five-chamber view, mitral flow was recorded using pulsed Doppler at the level of the tip of the mitral valves.

### Results:

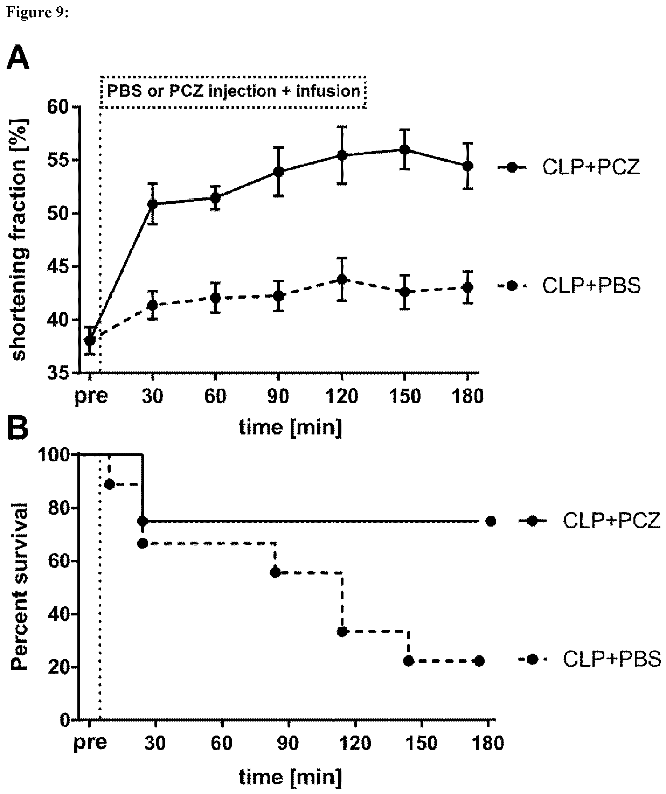

The sepsis-induced heart failure rats treated with PBS (CLP+PBS) show reduced shortening fraction compared to the sham animals (Fig. 9A). The CLP+PBS group also displays high mortality rate (Fig. 9B). In contrast, application of Procizumab to sepsis-induced heart failure rats improves shortening fraction (Fig. 9A) and drastically reduces the mortality rate (Fig. 9B).

### Example 7 - Effect of Procizumab on heart and kidney function

The effect of Procizumab in isoproterenol-induced heart failure in mice was studied by monitoring the shortening fraction and renal resistive index.

### Isoproterenol-induced cardiac stress in mice:

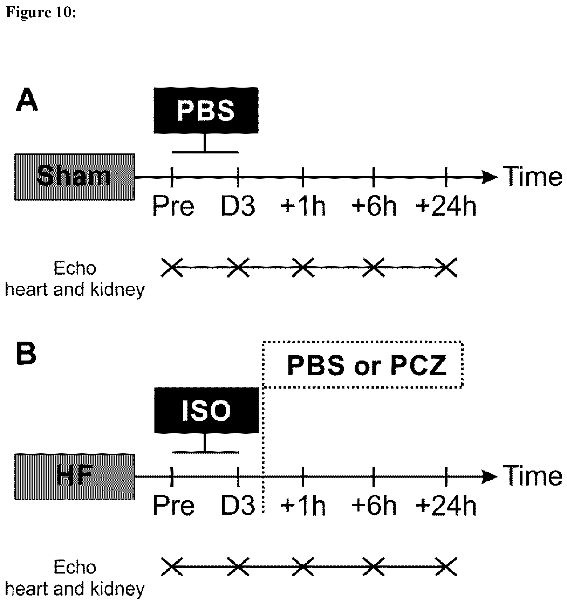

Acute heart failure was induced in male mice at 3 months of age by two daily subcutaneous injections of 300 mg/kg of Isoproterenol, a non-selective β-adrenergic agonist (DL-Isoproterenol hydrochloride, Sigma Chemical Co) (ISO) for two days (*Vergaro et al, 2016).* The ISO dilution was performed in NaCl 0.9%. Isoproterenol-treated mice were randomly assigned to two groups (Table 8) and PBS or Procizumab (10 mg/kg) were injected intravenously after baseline echocardiography (Gao et al., 2011) and renal resistive index measurements (*Lubas et al., 2014, Dewitte et al, 2012*) were performed at day 3 (Figure 10 A and B).

Cardiac function was assessed by echocardiography (*Gao et al., 2011*) and by the renal resistive index (*Lubas et al., 2014, Dewitte et al, 2012*) at 1 hour, 6 hours and 24 hours (Figure 10 A and B). The group of mice that was injected with vehicle (PBS) instead of isoproterenol was subjected to no further pharmacological treatment and served as the control group (Table 8).

**Table 8: list of experimental groups**

| **Group** | **Number of Animals** | **Treatment** |
|---|---|---|
| Sham+PBS | 27 | PBS |
| HF+PBS | 15 | PBS |
| HF+PCZ | 20 | PCZ |

### Results:

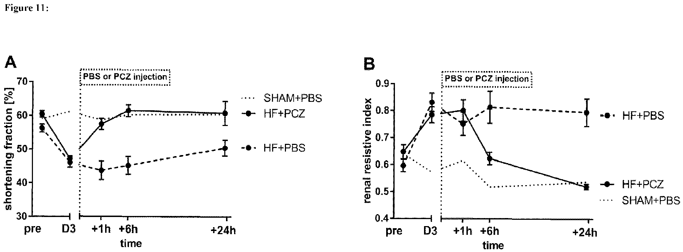

Application of Procizumab to isoproterenol-induced heart failure mice restores heart function within the first hour after administration (Fig. 11A). Kidney function of sick mice shows significant improvement at 6 hours post PCZ injection and is comparable to the kidney function of sham animals at 24 hours (Fig. 11B).

### Example 8 - Effect of Valsartan

The effect of an antagonist for the type I angiotensin II receptor (ATR1), Valsartan, in healthy mice injected with DPP3 was studied by monitoring the shortening fraction.

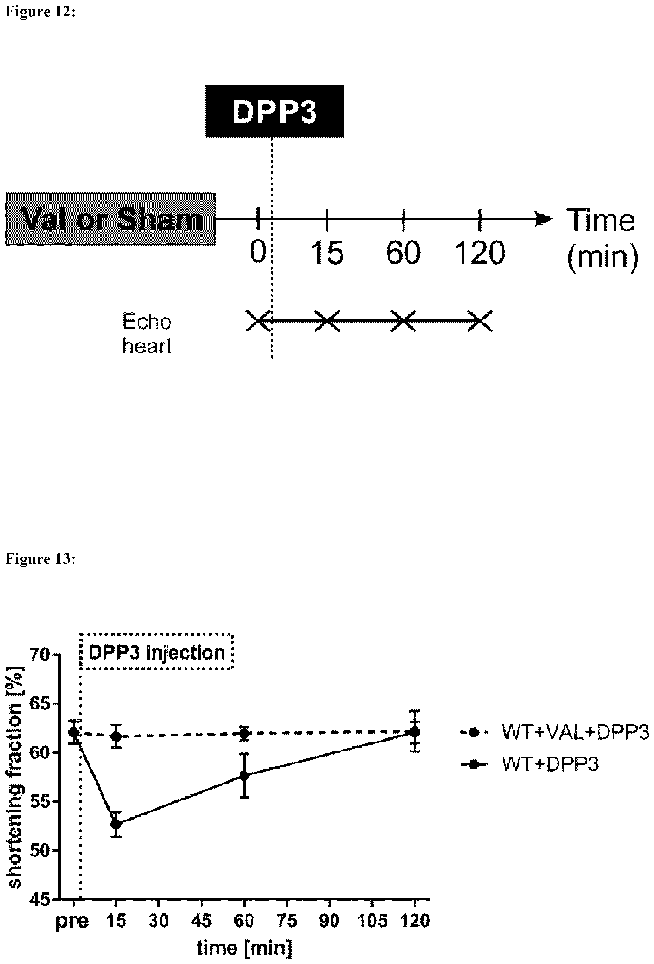

In this experiment, healthy Black 6 mice (8-12 weeks, group size refer to table 9) consumed water with 50 mg/kg Valsartan per day or just water (Table 9) for a period of two weeks. Subsequently, both groups received an intravenous injection of native DPP3 (600 µg/kg) and the shortening fraction was assessed according to Gao et al., 2011 at 15, 60 and 120 minutes (Figure 12).

**Table 9: list of experiment groups**

| **Group** | **Number of Animals** | **Treatment** |
|---|---|---|
| WT + DPP3 | 4 | Water + PBS / DPP3 injection |
| Val + DPP3 | 3 | Water + Valsartan / DPP3 injection |

### Results:

DPP3 injection to healthy mice lead to a significant decrease in the shortening fraction (Figure 13). In contrast, healthy mice treated with the angiotensin II receptor antagonist, Valsartan, and then submitted to DPP3 injection, showed no signs of heart dysfunction assessed by the shortening fraction. Therefore, the cardiac function is restored by the Valsartan treatment and thus the DPP3-mediated heart dysfunction is angiotensin II mediated.

The animals that were treated with Valsartan for two weeks have been adapted to blocking of the type I angiotensin II receptor, to the subsequent inhibited angiotensin II-mediated signaling and the inhibited AngII-mediated activity of the heart function. Apparently, under Valsartan treatment, the organism switched to other ways for activating cardiac function independent of type I angiotensin II receptor signaling, as this angiotensin signaling system has been inhibited by Valsartan.

When DPP3 cleaves Ang II and thus inhibits the angiotensin II-mediated activity of the heart function, those animals that are adapted to a down-regulated angiotensin-system (Valsartan treated animals) showed no signs of heart dysfunction as assessed by the shortening fraction. In contrast thereto, animals that were not treated with the angiotensin II receptor antagonist Valsartan and were not adapted to an inhibited Ang II-mediated signaling, showed a significant decrease in the shortening fraction in response to DPP3 injection and subsequent cleavage and inactivation of AngII
This experiment clearly shows the relationship between DPP3 and angiotensin II meaning that the DPP3-induced heart dysfunction is angiotensin II-mediated.

### Example 9 - DPP3 and organ dysfunction in sepsis

The same study as described in Example 2 (AdrenOSS-1) was used to assess the association between circulating DPP3 (cDPP3), organ (e.g. cardiovascular and renal dysfunction) in patients admitted for sepsis and septic shock. The AdrenOSS-1 is a European prospective, observational, multinational study (ClinicalTrials.gov NCT02393781) including 583 patients admitted to the ICU with sepsis or septic shock. The primary outcome (as described in example 2) was 28-day mortality. Secondary outcomes included organ failure defined by SOFA score, organ support with focus on vasopressor use and need for renal replacement therapy. Blood for the central laboratory was sampled within 24 hours after ICU admission and on day 2.

For the quantification of DPP3 protein concentrations (DPP3-LIA) an assay as recently described was used (Rehfeld et al. 2019. JALM 3(6): 943-953)*.*

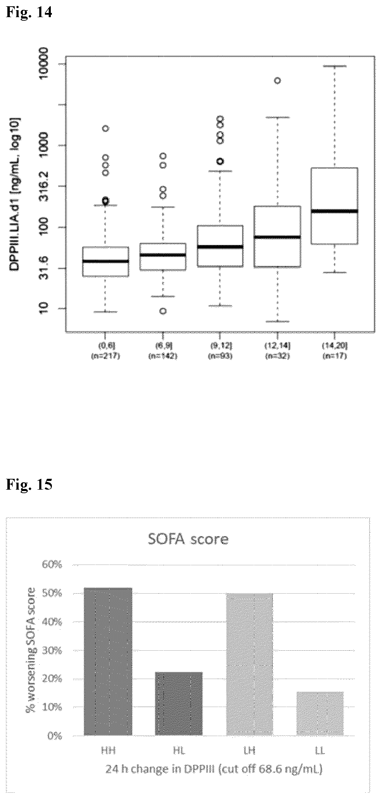

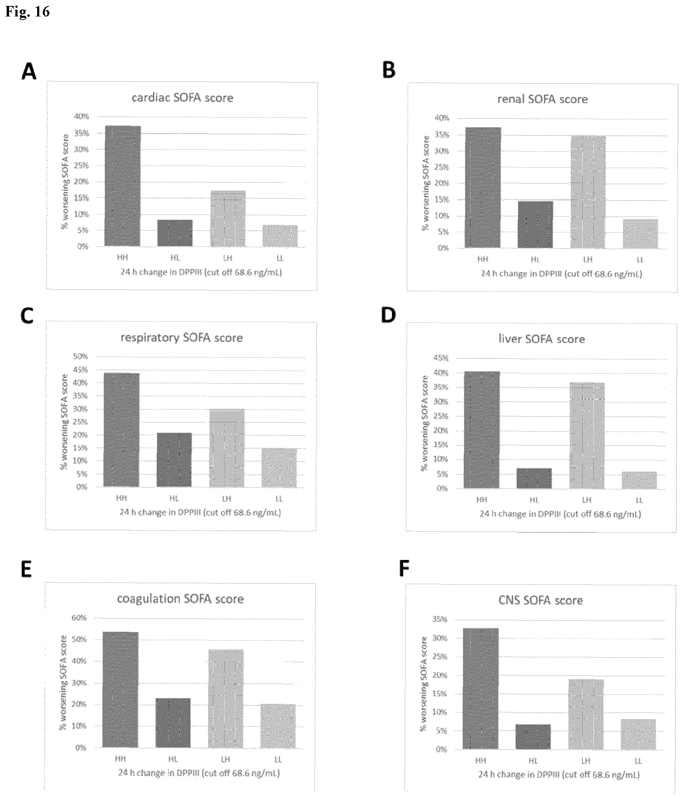

Median cDPP3 measured at admission in all AdrenOSS-1 patients was 45.1 ng/mL (inter quartile range 27.5-68.6). High DPP3 levels measured at admission were associated with worse metabolic parameters, renal and cardiac function and SOFA score: patients with DPP3 levels below the median had a median SOFA score (points) of 6 (IQR 4-9) compared to a median SOFA score of 8 (IQR 5-11) for patients with DPP3 levels above the median of 45.1 ng/mL (Fig. 14)
Whatever levels of cDPP3 at admission, high concentrations of cDPP3 levels 24 hours later were associated with worst SOFA scores whether global Fig. 15 or by organ (Fig. 16 A-F).

In summary these data showed that high levels of cDPP3 were associated with survival and the extent of organ dysfunction in a large international cohort septic or septic shock patients. The study found marked association between cDPP3 < 45.1 ng/ml at admission and short-term survival as well as the prognostic cut-off value of 45.1 pg/ml in both sepsis and septic shock. Concerning organ dysfunction, there was a positive relationship between cDPP3 and SOFA score at ICU admission. More importantly, the relationship between cPDPP3 levels and extent of organ dysfunction, seen at ICU admission, was also true during the recovery phase. Indeed, patients with high cDPP3 levels at admission who showed a decline towards normal cDPP3 values at day 2 were more likely to recover all organ function including cardiovascular, kidney, lung, liver.

### Example 10 - DPP3 in patients infected with coronavirus (SARS-CoV-2)

Plasma samples from 12 patients that were diagnosed of being infected with coronavirus (SARS-CoV-2) were screened for DPP3 and other biomarkers.

An immunoassay (LIA) or an activity assays (ECA) detecting the amount of human DPP3 (LIA) or the activity of human DPP3 (ECA), respectively, was used for determining the DPP3 level in patient plasma as described recently (Rehfeld et al. 2019. JALM 3(6): 943-953)*.*

Bio-ADM levels were measured using an immunoassay as described in Weber et al. 2017 ((Weber et al. 2017. JALM 2(2): 222-233).

The respective DPP3 and bio-ADM concentrations in individual samples are summarized in table 10.

**Table 10: DPP3 levels in samples from patients infected with coronavirus (SARS-CoV-2)**

| **Patient No.** | **DPP3 (ng/ml)** | **bio-ADM (pg/ml)** |
|---|---|---|
| 1 | 56 | 133 |
| 2 | 30 | 45 |
| 3 | 70 | 214 |
| 4 | 150 | 85 |
| 5 | 290 | 437 |
| 6 | 87 | 66 |
| 7 | 975 | 79 |
| 8 | 333 | 174 |
| 9 | 216 | 35 |
| 10 | 539 | 199 |
| 11 | 27 | 53 |
| 12 | 162 | 401 |
| **Median** | **156.0** | **109.0** |
| **mean** | **244.6** | **160.1** |

DPP3 concentrations ranged between 27 and 975 ng/ml with a median (IQR) of 156 (59.5 - 322.3) ng/ml. bio-ADM concentrations ranged between 35 and 437 pg/ml with a median (IQR) of 109 (56 - 210) pg/ml. DPP3 concentrations are significantly elevated compared to healthy subjects. Samples from 5,400 normal (healthy) subjects (swedish single-center prospective population-based Study (MPP-RES)) have been measured: median (interquartile range) plasma DPP3 was 14.5 ng/ml (11.3 ng/ml - 19 ng/ml). Median plasma bio-ADM (mature ADM-NH₂) in samples from (healthy) subjects was 24.7 pg/ml, the lowest value 11 pg/ml and the 99^{th} percentile 43 pg/ml *(*Marino et al. 2014. Critical Care 18:R34*).*

### SEQUENCES

**SEQ ID No. 1 - hDPP3 aa 1-737** 
MADTQYILPNDIGVSSLDCREAFRLLSPTERLYAYHLSRAAWYGGLAVLLQTSPEAP
YIYALLSRLFRAQDPDQLRQHALAEGLTEEEYQAFLVYAAGVYSNMGNYKSFGDTK
FVPNLPKEKLERVILGSEAAQQHPEEVRGLWQTCGELMFSLEPRLRHLGLGKEGITTY
FSGNCTMEDAKLAQDFLDSQNLSAYNTRLFKEVDGEGKPYYEVRLASVLGSEPSLDS
EVTSKLKSYEFRGSPFQVTRGDYAPILQKVVEQLEKAKAYAANSHQGQMLAQYIESF
TQGSIEAHKRGSRFWIQDKGPIVESYIGFIESYRDPFGSRGEFEGFVAVVNKAMSAKFE
RLVASAEQLLKELPWPPTFEKDKFLTPDFTSLDVLTFAGSGIPAGINIPNYDDLRQTEG
FKNVSLGNVLAVAYATQREKLTFLEEDDKDLYILWKGPSFDVQVGLHELLGHGSGK
LFVQDEKGAFNFDQETVINPETGEQIQSWYRSGETWDSKFSTIASSYEECRAESVGLY
LCLHPQVLEIFGFEGADAEDVIYVNWLNMVRAGLLALEFYTPEAFNWRQAHMQARF
VILRVLLEAGEGLVTITPTTGSDGRPDARVRLDRSKIRSVGKPALERFLRRLQVLKSTG
DVAGGRALYEGYATVTDAPPECFLTLRDTVLLRKESRKLIVQPNTRLEGSDVQLLEY
EASAAGLIRSFSERFPEDGPELEEILTQLATADARFWKGPSEAPSGQA
**SEQ ID No. 2 - hDPP3 aa 474-493 (N-Cys) - immunization peptide with additional N-terminal Cystein**
   CETVINPETGEQIQSWYRSGE
**SEQ ID No. 3 - hDPP3 aa 477-482 - epitope of AK1967**
   INPETG
**SEQ ID No. 4 - hDPP3 aa 480-483**
   ETGE
**SEQ ID No. 5 - variable region of murine AK1967 in heavy chain** 
QVTLKESGPGILQPSQTLSLTCSFSGFSLSTSGMSVGWIRQPSGKGLEWLAHIWWNDN
KSYNPALKSRLTISRDTSNNQVFLKIASVVTADTGTYFCARNYSYDYWGQGTTLTVS
S
**SEQ ID No. 6 - variable region of murine AK1967 in light chain** 
DVVVTQTPLSLSVSLGDPASISCRSSRSLVHSIGSTYLHWYLQKPGQSPKLLIYKVSNR
FSGVPDRFSGSGSGTDFTLKISRVEAEDLGVYFCSQSTHVPWTFGGGTKLEIK
**SEQ ID No. 7 - CDR1 of murine AK1967 in heavy chain**
   GFSLSTSGMS
**SEQ ID No. 8 - CDR2 of murine AK1967 in heavy chain**
   IWWNDNK
**SEQ ID No. 9 - CDR 3 of murine AK1967 in heavy chain**
   ARNYSYDY
**SEQ ID No. 10 - CDR1 of murine AK1967 in light chain**
   RSLVHSIGSTY
CDR2 of murine AK1967 in light chain
   KVS
SEQ ID No. 11 - CDR3 of murine AK1967 in light chain
   SQSTHVPWT
**SEQ ID No. 12 - humanized AK1967 - heavy chain sequence (IgG1κ backbone)** 
MDPKGSLSWRILLFLSLAFELSYGQITLKESGPTLVKPTQTLTLTCTFSGFSLSTSGMS
VGWIRQPPGKALEWLAHIWWNDNKSYNPALKSRLTITRDTSKNQVVLTMTNMDPV
DTGTYYCARNYSYDYWGQGTLVTVSSASTKGPSVFPLAPSSKSTSGGTAALGCLVK
DYFPEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHEALH
NHYTQKSLSLSPG
**SEQ ID No. 13 - humanized AK1967 - light chain sequence (IgG1κ backbone)**

METDTLLLWVLLLWVPGSTGDIVMTQTPLSLSVTPGQPASISCKSSRSLVHSIGSTYL
YWYLQKPGQSPQLLIYKVSNRFSGVPDRFSGSGSGTDFTLKISRVEAEDVGVYYCSQS
THVPWTFGGGTKVEIKRTVAAPSVFIFPPSDEQLKSGTASVVCLLNNFYPREAKVQW
KVDNALQSGNSQESVTEQDSKDSTYSLSSTLTLSKADYEKHKVYACEVTHQGLSSPV
TKSFNRGEC

## Claims

1. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus, the method comprising:
• determining the level of dipeptidyl peptidase 3 (DPP3) in a sample of bodily fluid of said patient,
• comparing said level of determined DPP3 to a pre-determined threshold, and
• correlating said level of determined DPP3 with the risk of life-threatening deterioration or an adverse event, or
• correlating said level of determined DPP3 with the severity, or
• correlating said level of determined DPP3 with the success of a therapy or intervention.
• correlating said level of DPP3 with a certain therapy or intervention, or
• correlating said level of DPP3 with the management of said patient.

2. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to claim 1, wherein said coronavirus is selected from the group comprising SARS-CoV-1, SARS-CoV-2, MERS-CoV, in particular SARS-CoV-2

3. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to claim 1 or 2, wherein said adverse event is selected from the group comprising death, organ dysfunction, and shock.

4. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to claims 1 to 3, wherein said level of determined DPP3 is above a pre-determined threshold.

5. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to claims 1 to 4, wherein the level of DPP3 is determined by contacting said sample of bodily fluid with a capture binder that binds specifically to DPP3.

6. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to claims 1 to 5, wherein said determination comprises the use of a capture-binder that binds specifically to full-length DPP3 wherein said capture-binder may be selected from the group of antibody, antibody fragment or non-IgG scaffold.

7. A method for (a) diagnosing or predicting the risk of life-threatening deterioration or an adverse event or (b) diagnosing or prognosing the severity or (c) predicting or monitoring the success of a therapy or intervention or (d) therapy guidance or therapy stratification or (e) patient management in a patient infected with a coronavirus according to claims 1 to 6, wherein said patient is treated with an inhibitor of DPP3 activity and/or an angiotensin-receptor-agonist and/ or a precursor of said angiotensin-receptor-agonist.

8. Inhibitor of the activity of DPP3 and/or an angiotensin-receptor-agonist and/ or a precursor of said angiotensin-receptor-agonist for use in therapy or intervention in a patient infected with a coronavirus.

9. Inhibitor of the activity of DPP3 and/or an angiotensin-receptor-agonist and/ or a precursor of said angiotensin-receptor-agonist for use in therapy or intervention in a patient infected with a coronavirus according to claim 8 wherein said coronavirus is selected from the group comprising Sars-CoV-1, Sars-CoV-2, MERS-CoV, in particular Sars-CoV-2.

10. Inhibitor of the activity of DPP3 and/or an angiotensin-receptor-agonist and/ or a precursor of said angiotensin-receptor-agonist for use in therapy or intervention in a patient infected with a coronavirus according to claim 18 or 19 wherein said patient has a level of DPP3 in a sample of bodily fluid of said subject that is above a predetermined threshold when determined by a method according to any of claims 1 - 7.

11. Inhibitor of the activity of DPP3 for use in therapy or intervention in a patient infected with a coronavirus according to claims 8 - 10, wherein the inhibitor of the activity of DPP3 is selected from the group comprising anti-DPP3 antibody or anti-DPP3 antibody fragment or anti-DPP3 non-Ig scaffold.

12. Inhibitor of the activity of DPP3 for use in therapy or intervention in a patient infected with a coronavirus according to claims 8 - 11, wherein said inhibitor is an anti-DPP3 antibody or anti-DPP3 antibody fragment or anti-DPP3 non-Ig scaffold that binds an epitope of at least 4 to 5 amino acids in length comprised in SEQ ID No. 1.

13. Inhibitor of the activity of DPP3 for use in therapy or intervention in a patient infected with a coronavirus according to claims 8 - 12, wherein said inhibitor is an anti-DPP3 antibody or anti-DPP3 antibody fragment or anti-DPP3 non-Ig scaffold that binds an epitope of at least 4 to 5 amino acids in length comprised in SEQ ID No. 2.

14. Inhibitor of the activity of DPP3 for use in therapy or intervention in a patient infected with a coronavirus according to claims 8 - 13, wherein said antibody is a monoclonal antibody or monoclonal antibody fragment.

15. Inhibitor of the activity of DPP3 for use in therapy or intervention in a patient infected with a coronavirus according to claim 14, wherein the complementarity determining regions (CDR's) in the heavy chain comprises the sequences:
SEQ ID NO.: 7, SEQ ID NO.: 8 and/ or SEQ ID NO.: 9
and the complementarity determining regions (CDR's) in the light chain comprises the sequences:
SEQ ID NO.: 10, KVS and/or SEQ ID NO.: 11.

16. Inhibitor of the activity of DPP3 for use in therapy or intervention in a patient infected with a coronavirus according to claim 15, wherein said monoclonal antibody or antibody fragment is a humanized monoclonal antibody or humanized monoclonal antibody fragment.

17. Inhibitor of the activity of DPP3 for use in therapy or intervention in a patient infected with a coronavirus according to claim 16, wherein the heavy chain comprises the sequence:
SEQ ID NO.: 12
and wherein the light chain comprises the sequence:
SEQ ID NO.: 13.

18. Angiotensin-receptor-agonist and/ or a precursor thereof for use in therapy or intervention in a patient infected with a coronavirus according to claims 8 to 17, wherein said Angiotensin-receptor-agonist and/ or a precursor thereof is selected from the group comprising angiotensin I, angiotensin II, angiotensin III, angiotensin IV.
